# EUROPEAN PATENT APPLICATION

(11) **EP 3 061 762 A1**
(43) Date of publication of application: **31.08.2016**
(21) Application number: 15156740.1
(22) Date of filing: 26.02.2015
(51) Int. Cl.: C07D 487/22

(54) **PHOTOSENSITIZERS**

(71) Applicant: Universiteit van Amsterdam, 1012 WX Amsterdam (NL)
(72) Inventor: Chen, Hung-Cheng, 1098 XH Amsterdam (NL); Brouwer, Albert Manfred, 1098 XH Amsterdam (NL)
(74) Representative: Hesselink, Dinah Elisabeth

(57) **Abstract**

The present invention relates to a photosensitizer comprising a porphyrin compound of the formula (I): 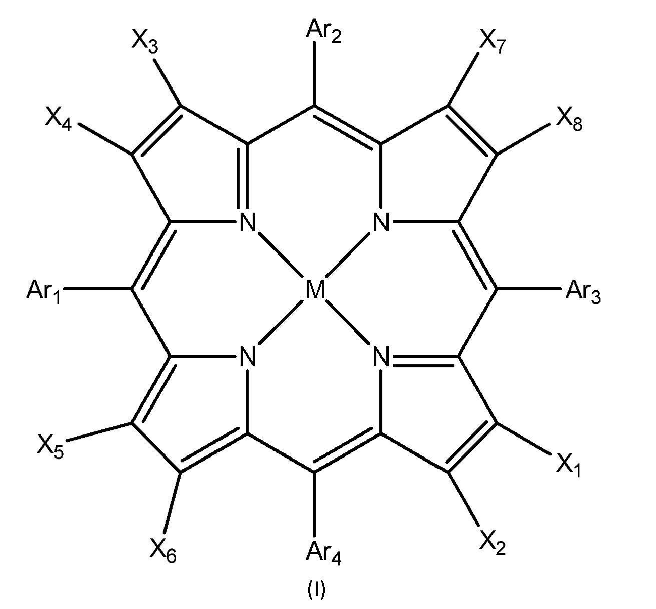 wherein:
Ar₁, Ar₂, Ar₃ and Ar₄ are each independently selected from aryl or heteroaryl, wherein each of said aryl or heteroaryl substituents may be optionally substituted,
X₁, X₂, X₃, and X₄ are each independently selected from H or halogen, whereby at least one of X₁, X₂, X₃, and X₄ is halogen;
X₅ and X₆ are each independently selected from H, hydrocarbyl or a halogen-containing group, or are linked together form an aromatic ring;
X₇ and X₈ are each independently selected from H , hydrocarbyl or a halogen-containing group, or are linked together form an aromatic ring; and
M is a metal, P or Si.

## Description

The present invention relates to a photosensitizer. The present invention also relates to porphyrin compounds that may be used as photosensitizers.

### BACKGROUND

The harnessing of solar energy to generate a source of fuel provides a potential solution to the increasing energy requirements of the global population. One option for fuel production is light-driven water splitting to produce O₂ and H₂ by artificial photosynthesis.

Visible-light-driven water oxidation may be carried out using a system comprising three components: a water oxidation catalyst (WOC), a photosensitizer, and a sacrificial electron acceptor. On exposure to visible light, the photosensitizer absorbs photons, and the excited state generated following this absorption transfers an electron to the sacrificial electron acceptor. The resulting oxidized species activates the water oxidation catalyst from its low oxidation state to a higher one, which then catalyses the oxidation of water. An exemplary schematic illustrating this three-component system is shown below. In this example, the photosensitizer is denoted by PS and the sacrificial electron acceptor comprises S₂O₈²⁻ ions:

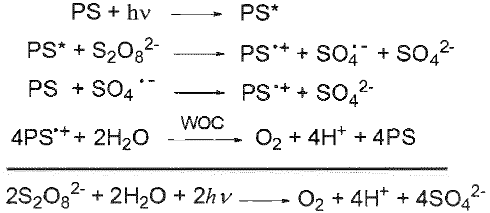

While various water oxidation catalysts are known, the availability of suitable photosensitizers for use in light-driven water oxidation is more limited. W. J. Youngblood, S.-H. A. Lee, Y. Kobayashi, E. A. Hernandez-Pagan, P. G. Hoertz, T. A. Moore, A. L. Moore, D. Gust, T. E. Mallouk, J. Am. Chem. Soc. 2009, 131, 926-927 describes the use of ruthenium polypyridine complex as photosensitizers. G. S. Nahor, P. Neta, P. Hambright, A. N. Thompson, A. Harriman, J. Phys. Chem. 1989, 93, 6181-6187 discusses the use of metalloporphyrins as photosensitizers. However, the use of these metalloporphyrins as photosensitizers is limited by the high oxidation potential required to provide sufficient electron transfer driving force to activate the water oxidation catalyst.

In order to perform visible light-driven water oxidation on a large-scale and long-term basis, it is desirable that the components used in the oxidation process are cost-effective and robust. For example, for certain applications, the use of readily available ("earth abundant") metals or other elements in the photosensitizer or water oxidation catalyst, or both, may be desirable. It may additionally be desirable to provide a photosensitizer with a highly positive oxidation potential. A high oxidation potential can provide an improved ability to photoactivate the water oxidation catalyst. It may also be desirable to provide a photosensitizer that is able to capture photons over a large part of the solar spectrum.

### DESCRIPTION

According to the present invention, there is provided a photosensitizer comprising a porphyrin compound of the formula (I): 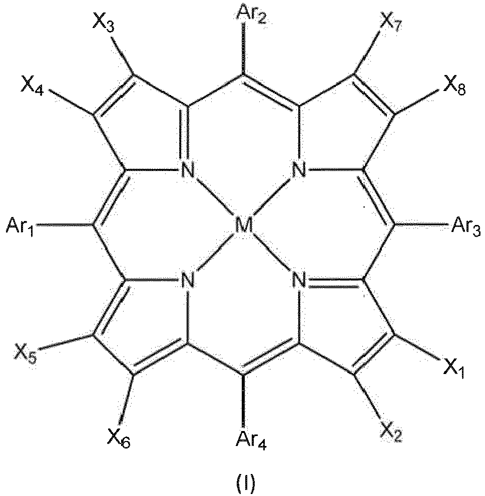 wherein:
Ar₁, Ar₂, Ar₃ and Ar₄ are each independently selected from aryl or heteroaryl,
X₁, X₂, X₃, and X₄ are each independently selected from H or halogen, whereby at least one of X₁, X₂, X₃, and X₄ is halogen;
X₅ and X₆ are each independently selected from H, hydrocarbyl or a halogen-containing group, or are linked together form an aromatic ring;
X₇ and X₈ are each independently selected from H, hydrocarbyl or halogen, or are linked together form an aromatic ring; and
M is a metal, P or Si.

The present inventors have found that, by halogenating the porphyrin ring at at least one of the X₁, X₂, X₃ and X₄ positions, it is possible to produce a macrocycle with improved photostability. Furthermore, by halogenating the porphyrin ring in this way, it may be possible to positively increase the oxidation potential of the molecule and/or extend the absorption spectrum of the molecule. Advantageously, the porphyrin ring may be halogenated at each of the X₁, X₂, X₃ and X₄ positions.

As noted above, X₁, X₂, X₃, and X₄ are each independently selected from H or halogen, whereby at least one of X₁, X₂, X₃, and X₄ is halogen. Preferably, 2 or more of X₁, X₂, X₃, and X₄ is halogen, for example, the same halogen. In a preferred embodiment, 3 or more of X₁, X₂, X₃, and X₄ is halogen, for example, the same halogen. In a most preferred embodiment, all of X₁, X₂, X₃, and X₄ is halogen, for example, the same halogen. Suitable halogens include F, Cl and Br. Preferably, the halogen is F or Cl.

In the compound (I), X₅ and X₆ are each independently selected from H, hydrocarbyl or a halogen-containing group, or are linked together form an aromatic ring. Similarly, X₇ and X₈ are each independently selected from H, hydrocarbyl or a halogen-containing group, or are linked together form an aromatic ring. Suitable hydrocarbyl groups include C₁- C₁₀ hydrocarbyl groups, for example, C₁ - C₁₀ alkyl groups (e.g. methyl, ethyl, propyl and butyl). Suitable halogen-containing groups include halogens and halogenated hydrocarbyl groups. Suitable halogens include F, Cl and Br. Suitable halogenated hydrocarbyl groups include halogenated alkyl groups, for instance, halogenated C₁-C₁₀ alkyl groups. Examples include -CₙX₂ₙ₊₁, where X is F, Cl, Br or I and n is an integer of 1 to 6. Preferably, X₅, X₆, X₇ and X₈ are each H; or X₅ and X₆ are linked together form an aromatic ring, and X₇ and X₈ are linked together form an aromatic ring. In one embodiment, all of X₁, X₂, X₃, and X₄ are halogen; and X₅, X₆, X₇ and X₈ are each H; or X₅ and X₆ are linked together form an aromatic ring, and X₇ and X₈ are linked together form an aromatic ring. A suitable aromatic ring comprises a phenyl ring that is fused to the pyrrole rings of the porphyrin. The aromatic ring may be a phenyl ring that is fused to the pyrrole rings of the porphyrin. Other examples include heteroaromatic rings or fused rings (e.g. naphthalene), for example, containing a phenyl ring that is fused to the pyrrole rings of the porphyrin.

In compound (I), Ar₁, Ar₂, Ar₃ and Ar₄ are each independently selected from aryl or heteroaryl. Suitable examples of aryl and heteroaryl groups include: Ar

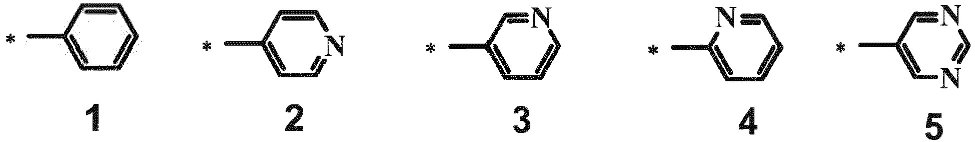

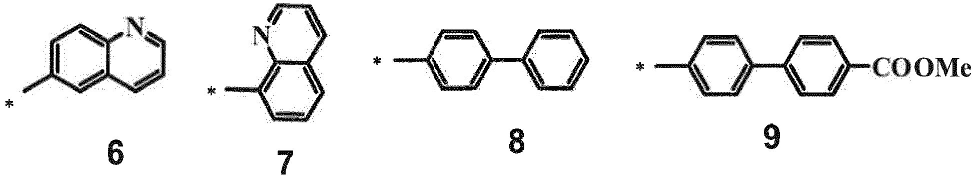

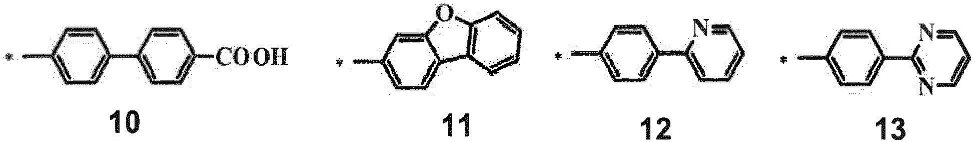

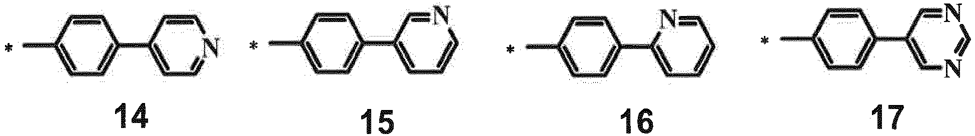

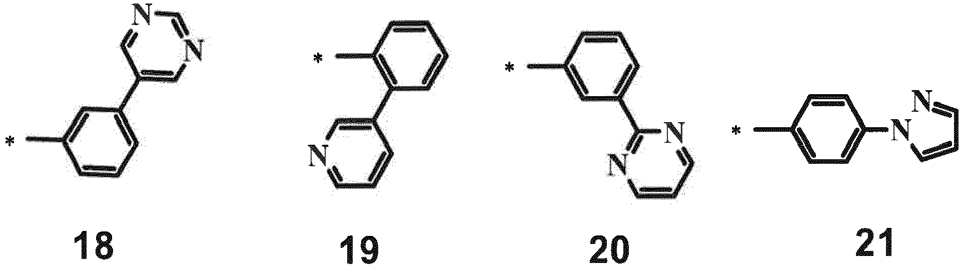

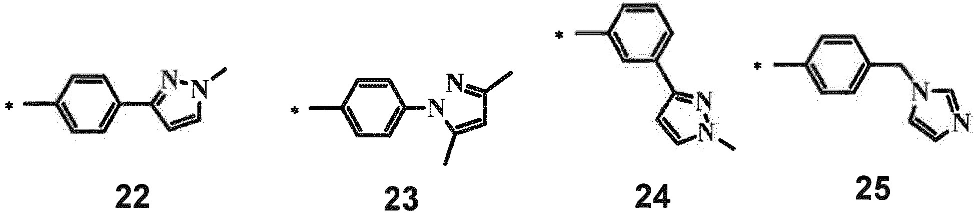

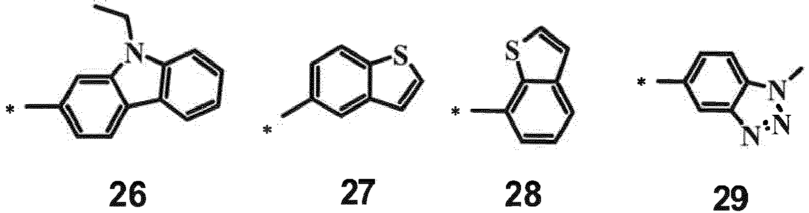

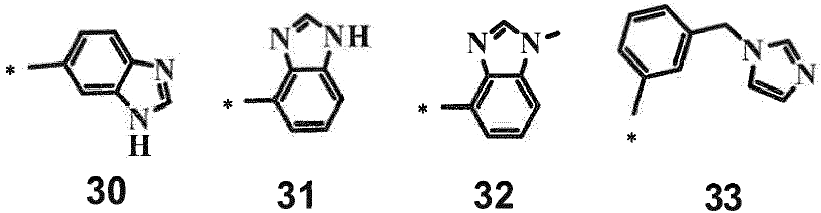

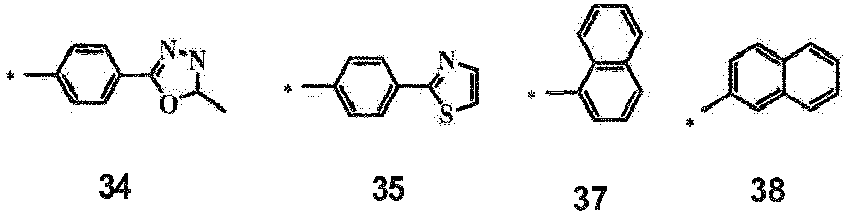

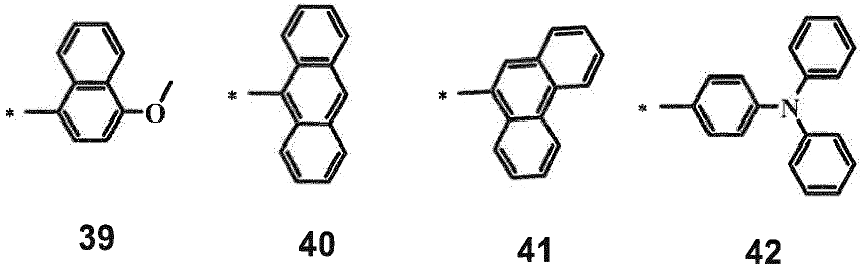

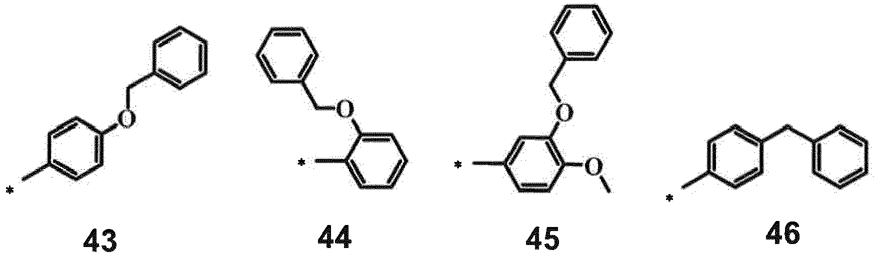

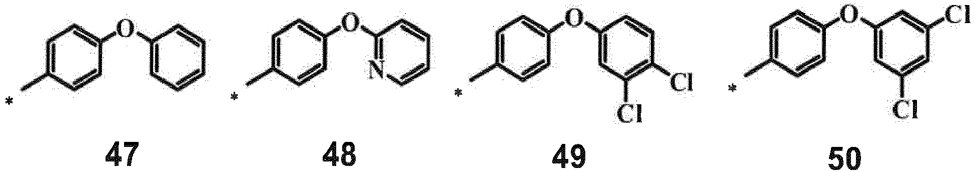

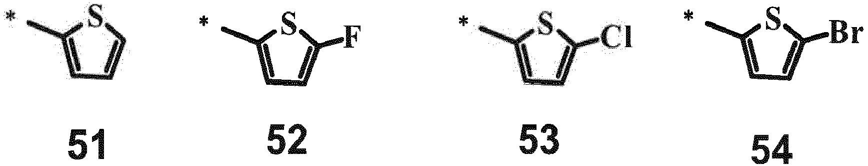

A preferred example of a suitable aryl group is a phenyl group. A preferred example of a suitable heteroaryl group is a pyridyl group.

In one embodiment, Ar₁, Ar₂, Ar₃ and Ar₄ are each independently selected from aryl or heteroaryl, wherein each of said aryl or heteroaryl substituent may be optionally substituted with a substituent comprising at least one halogen, hydrocarbyl, carboxylate, ketone, ether, ester, hydroxyl, sulphonate, sulphone, thioether, boron-containing group, amine, amide, nitrile, nitro, heteroalkyl, and phosphorus-containing group. Examples of suitable substituents for any one of Ar₁, Ar₂, Ar₃ and Ar₄ include: R

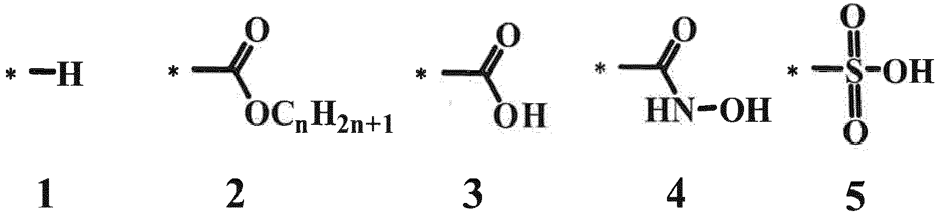

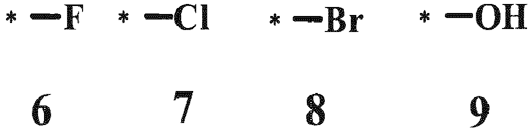

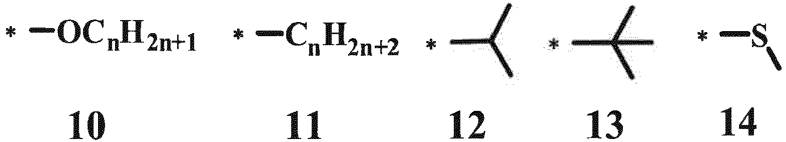

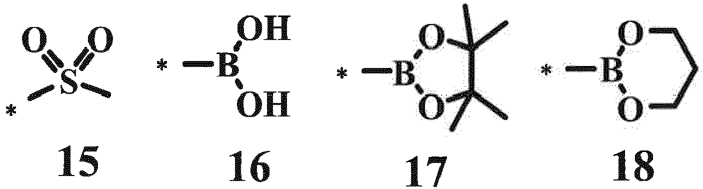

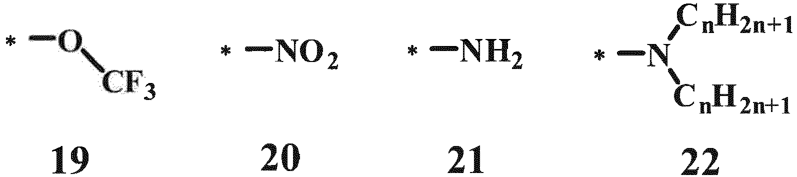

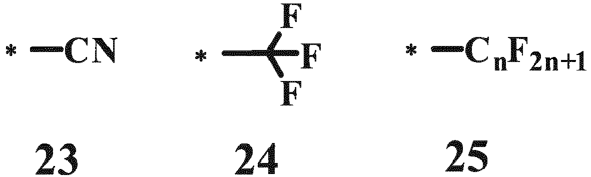

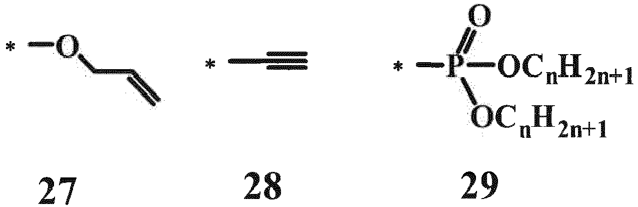

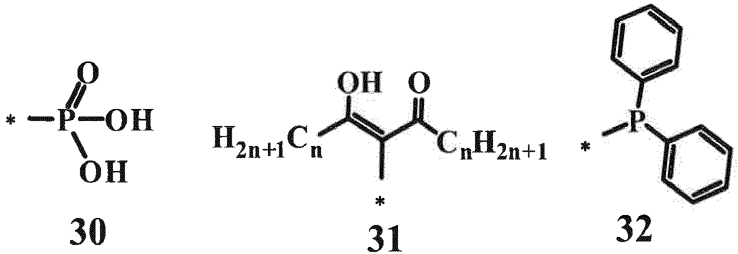

In one embodiment, Ar₁, Ar₂, Ar₃ and Ar₄ are each substituted with one or more substituents comprising at least one halogen, hydrocarbyl, carboxylate, ketone, ether, ester, hydroxyl, sulphonate, sulphone, thioether, boron-containing group, amine, amide, nitrile, nitro, heteroalkyl, and phosphorus-containing group. The substituent may be selected to improve the water-solubility of the compound (I). As described in further detail below, water-soluble embodiments of compound (I) may be dissolved in an electrolyte of a photoelectrochemical cell.

In a preferred embodiment, Ar₁, Ar₂, Ar₃ and Ar₄ are each independently selected from aryl or heteroaryl, wherein said aryl or heteroaryl may be optionally substituted with a carboxylate group. In one preferred embodiment, Ar₁, Ar₂, Ar₃ and Ar₄ are each phenyl or each pyridyl, said phenyl or pyridyl group being optionally substituted with one or more (preferably one) carboxylate group. In a preferred embodiment, compound (I) has the formula below: 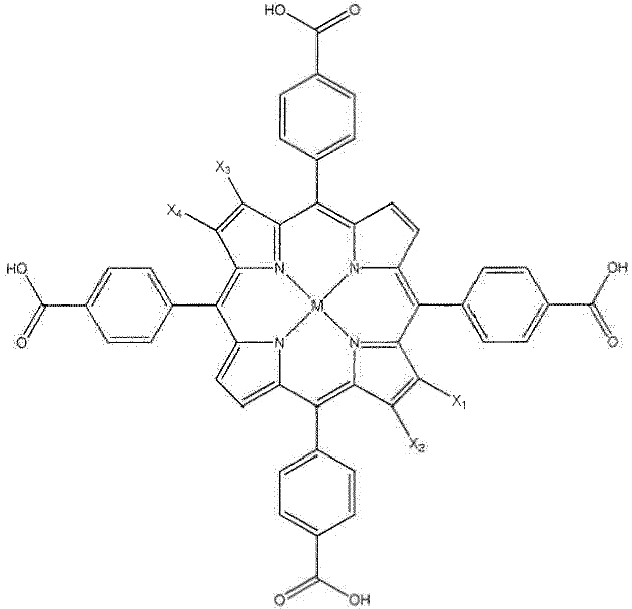 where X₁, X₂, X₃ and X₄ are each halogen, for example, F or Cl, preferably Cl. M is as defined above and is preferably Cu or Ni.

As noted above, M is a metal, P or Si. M may be selected from one of Cr, Ru, Ni, Mg, Zn, Co, Pd, Pt, Mn, Fe, Ag, Sn, Au, In, Ir, Ge, Pb, Al, Cu, P and Si. Advantageously, embodiments of the present invention allow more cost effective metals to be used to form photosensitizers. Thus, for example, M may be selected from Cu and Ni.

For the avoidance of doubt, M may be co-ordinated to one or more (e.g. two) additional ligands (not shown). Examples of such ligands include: Y

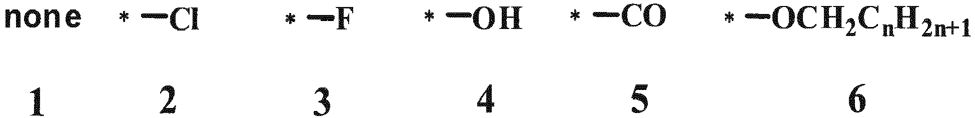

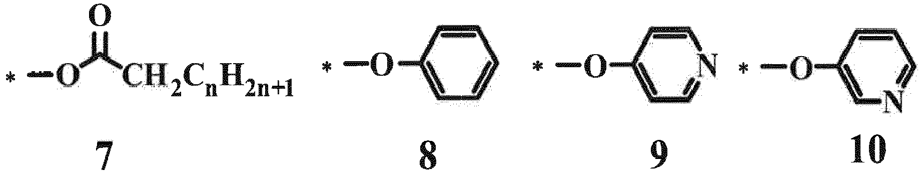

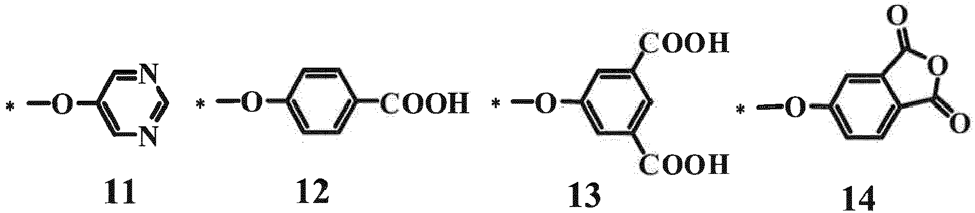

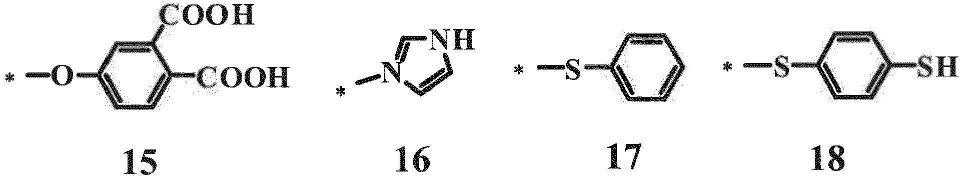

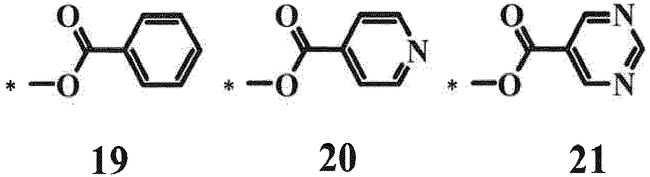

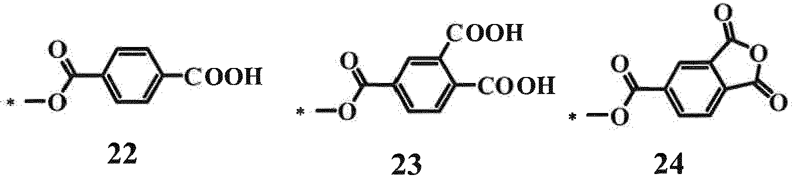

According to a further aspect of the present invention, there is provided a photoelectrochemical cell comprising a photosensitizer as defined herein. The cell may comprise a) an electrolyte comprising the compound of the formula (I) dissolved or dispersed therein or b) a photoelectrode comprising a compound of the formula (I). The photoelectrode may be a photoanode or a photocathode. Preferably, the photoelectrode is a photoanode. The photoanode may comprise:
(i) a current collector;
(ii) a semiconductor metal oxide coating,
(iii) a photosensitizer compound as described herein deposited on the semiconductor metal coating; and, optionally,
(iv) a water oxidation catalyst (WOC).

The cell above may be used in a method of oxidizing water to produce oxygen and hydrogen. The method may comprise: (i) providing a cell as described above; (ii) introducing water into the cell; and (iii) exposing the cell to light of a suitable wavelength.

The photosensitizer described herein may be used in a system for performing visible-light-driven water oxidation. The system may comprise (i) the photosensitizer as described herein; (ii) a water oxidation catalyst (WOC); and (iii) a sacrificial electron acceptor.

The present invention also provides a photoelectrode comprising a photosensitizer as described herein. For example, where the photoelectrode may comprise
(i) a current collector;
(ii) a semiconductor metal oxide coating,
(iii) a photosensitizer compound as described herein deposited on the semiconductor metal coating; and, optionally,
(iv) a water oxidation catalyst (WOC).

In yet another aspect of the present invention, there is provided a compound of the formula (I') or (I"): 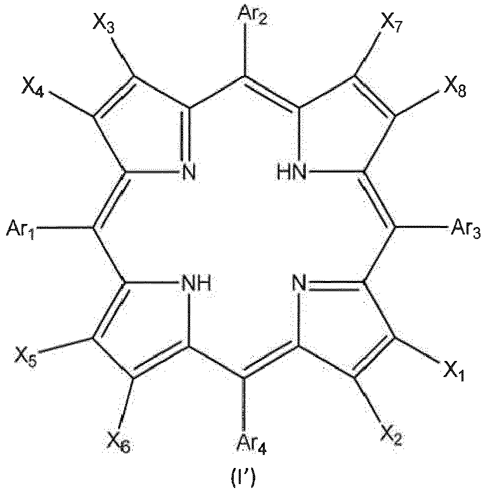 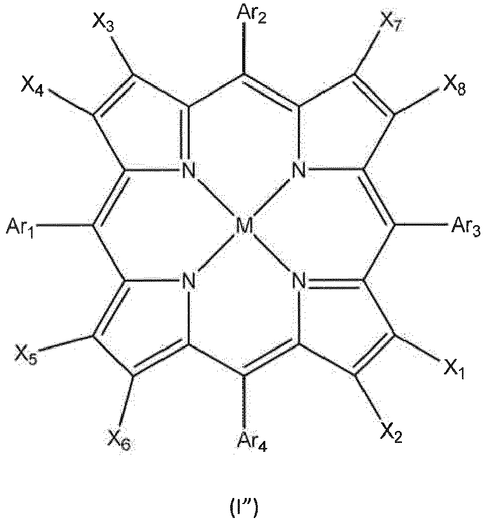 wherein:
Ar₁, Ar₂, Ar₃ and Ar₄ are each independently selected from aryl or heteroaryl, X₁, X₂, X₃, and X₄ are each halogen,
X₅, X₆, X₇ and X₈ are each H; or
X₅ and X₆ are linked together form an aromatic ring, and X₇ and X₈ are linked together form an aromatic ring, and
where present, M is a metal, P or Si.

In a preferred embodiment, the compound is of the formula below: 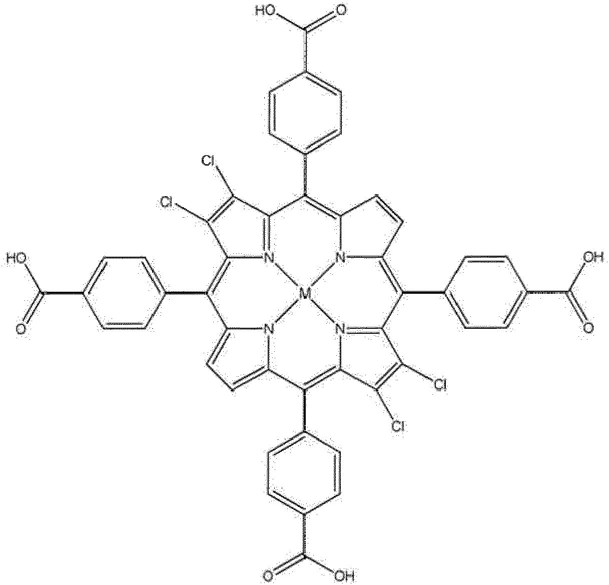 where M is preferably Cu or Ni.

### BRIEF DESCRIPTION OF THE DRAWINGS

Embodiments of the invention are further described hereinafter with reference to the accompanying drawings, in which:

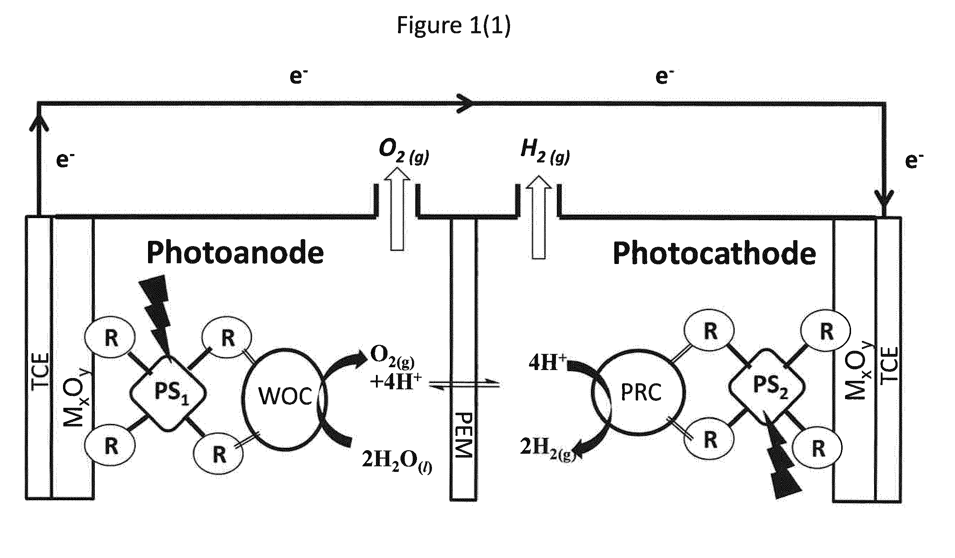

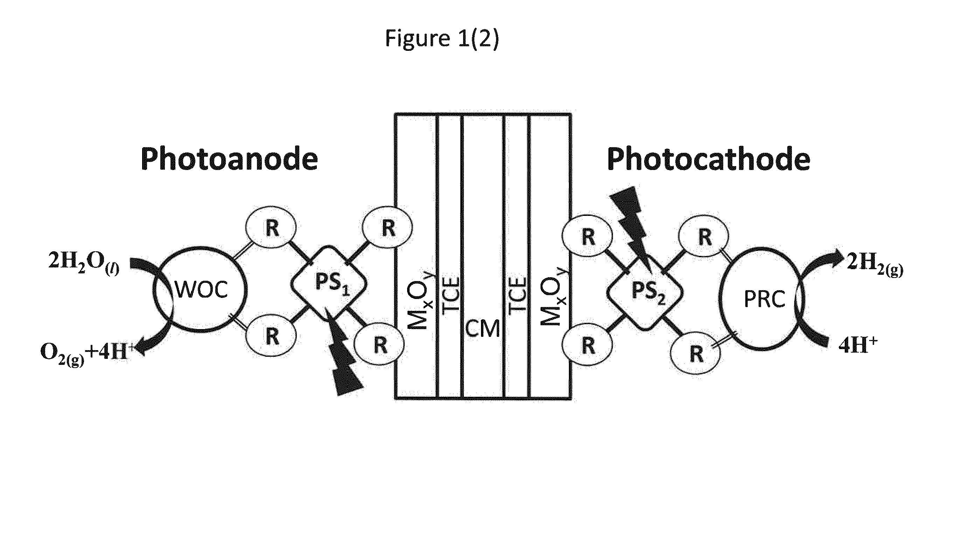

**Figure 1** is a schematic diagram of wired and wireless solar water-splitting dye-sensitized photoelectrochemical cells. In the photoanode, photosensitizers according to embodiments of the present invention can be immobilized on semiconductor metal oxide coating transparent conducting electrode and linked to the water-oxidation catalyst. In the photocathode, the porphyrin photosensitizers according to embodiments of the present invention can be also immobilized on semiconductor metal oxide coating transparent conducting electrode and linked to proton-reduction catalyst. The proton-exchange membrane is used to separate the photoanode and photocathode in the wired configuration. The conducting metal is used to combine the photoanode and photocathode together in the wireless configuration. In Figure 1, the following abbreviations are used: PS = photosensitizer, R= anchoring group, WOC = water-oxidation catalyst, PRC = proton-reduction catalyst, MₓO_{y} = semiconducting oxide, TCE = transparent conducting electrode, PEM= proton-exchange membrane, CM = conducting metal.

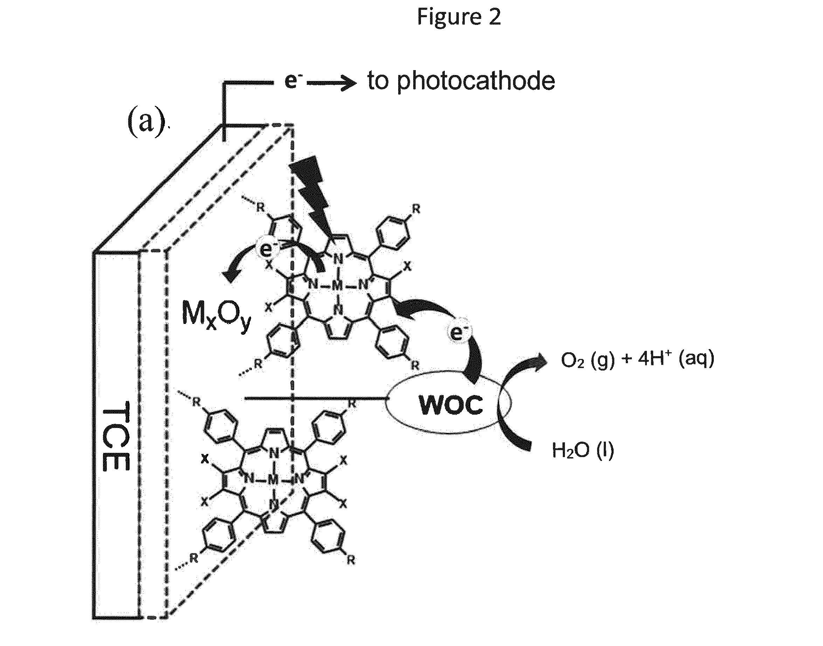

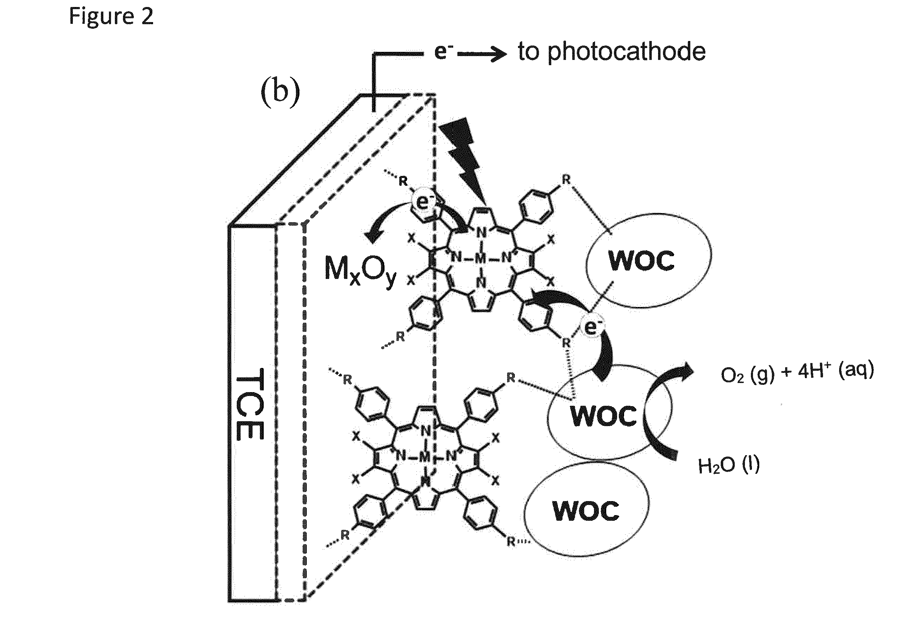

**Figure 2** is a schematic diagram of a dye-sensitized photoanode configuration according to one embodiment of the present invention in which the porphyrin photosensitizer and water-oxidation catalyst are applied by (a) co-deposition and (b) layer-by-layer stacking on the metal oxide semiconductor layer.

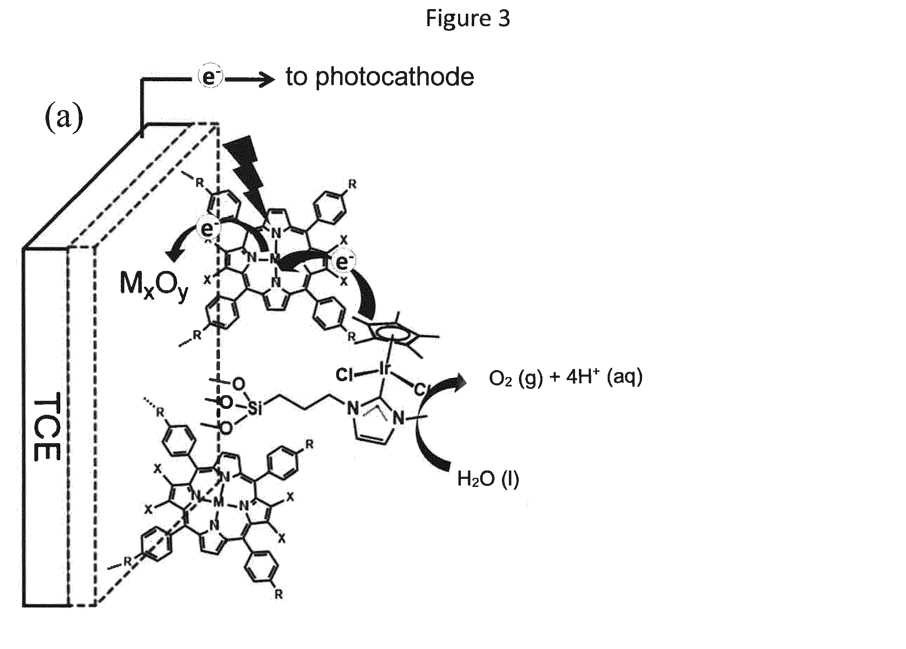

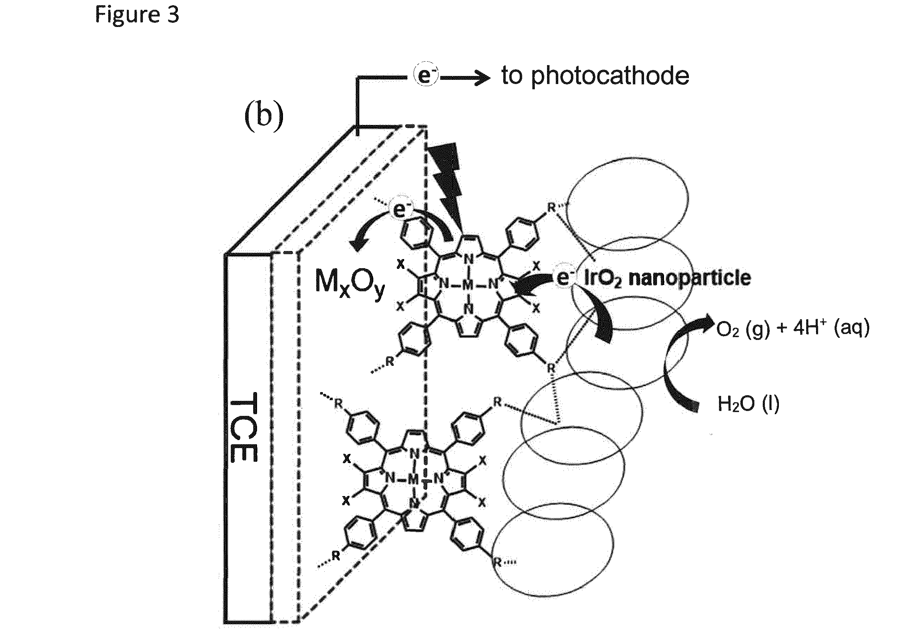

**Figure 3** is a schematic diagram of a dye-sensitized photoanode configuration according to one embodiment of the present invention in which the porphyrin photosensitizer and water-oxidation catalyst are applied to the metal oxide semiconductor layer by (a) co-deposition of an Ir-NHC WOC on top of a porphyrin layer and (b) co-deposition of an IrO₂ nanoparticle WOC on the top of a porphyrin layer.

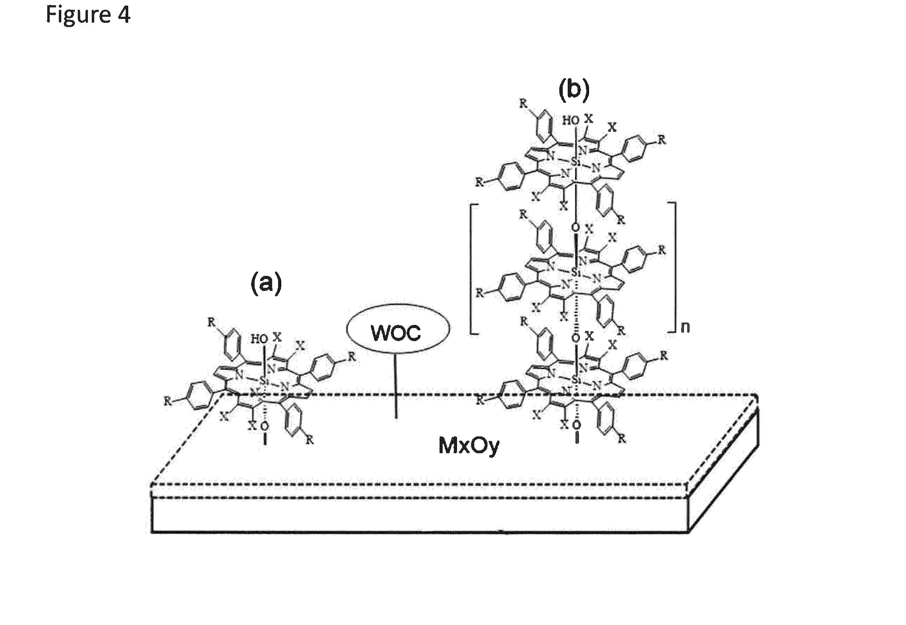

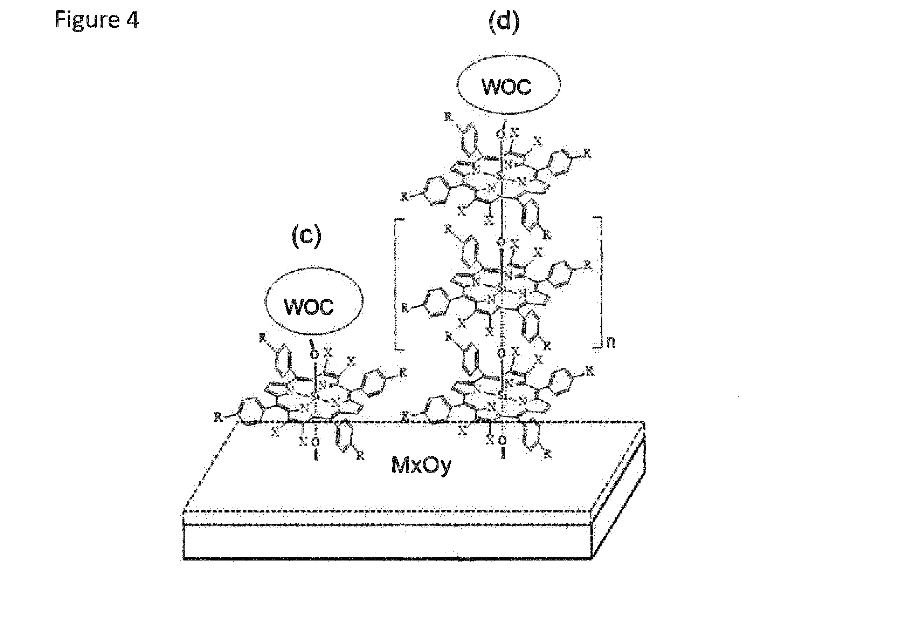

**Figure 4** is a schematic diagram of a dye-sensitized photoanode configuration according to one embodiment of the present invention comprising a silicon(IV)-porphyrin photosensitizer and water-oxidation catalyst; in which the photosensitizer and the WOC are applied by co-deposition ((a) and (b)) and layer-by-layer stacking ((c) and (d)) on the metal-oxide semiconductor layer.

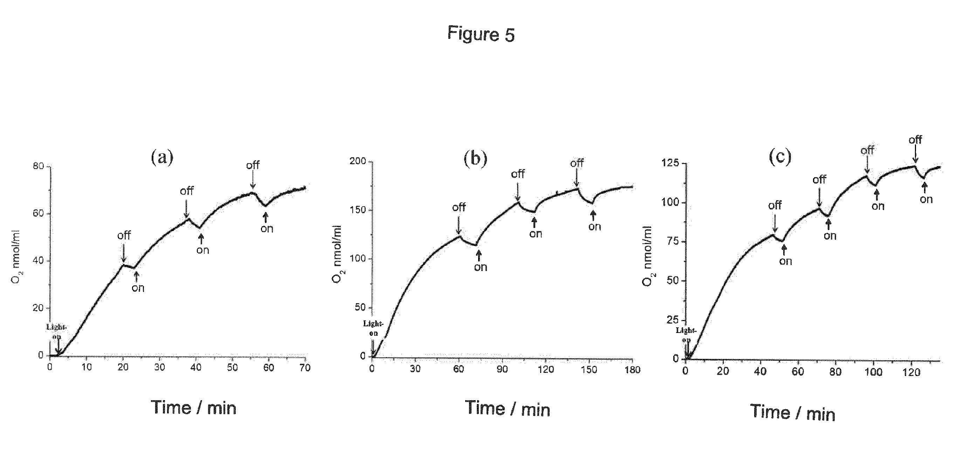

**Figure 5** shows the photochemical oxygen evolution in 1.5 mL of pH 7.0, 0.1 M KPi buffer solutions containing K₂S₂O₈ (5.0 × 10⁻² M) with (a) Cu(II)-TClTCPP (6.7 × 10⁻⁴ M) and Co(NO₃)₂ precatalyst (1.0 × 10⁻⁴ M), (b) Ni(II)-TClTCPP (6.7 × 10⁻⁴ M) and Co₄O₄ cubane (5.0 × 10⁻⁵ M). (c) Ni(II)-TClTCPP (6.7 × 10⁻⁴ M) and Co(NO₃)₂ precatalyst (1.0 × 10⁻⁴ M).

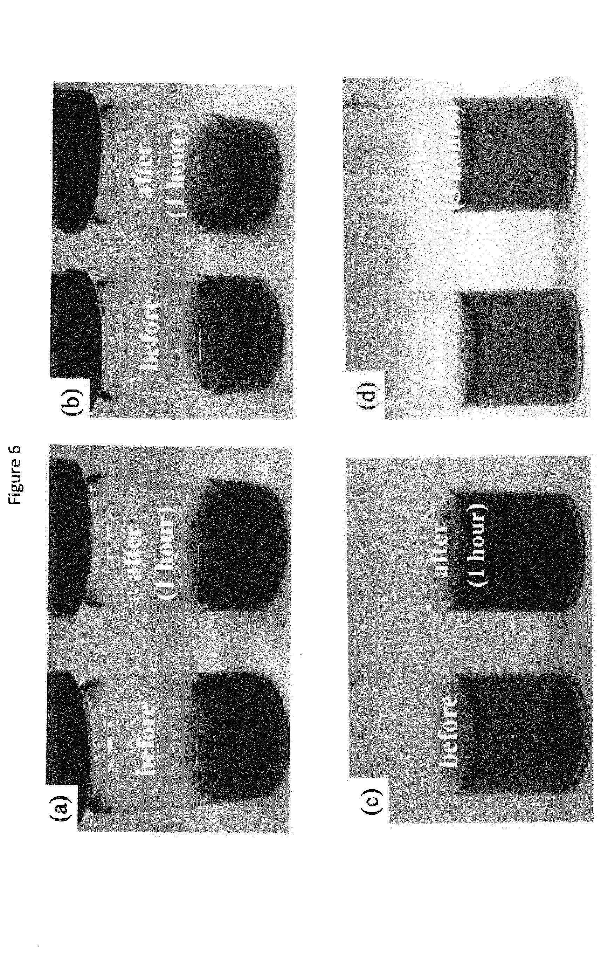

**Figure 6** provides photographs showing photocatalytic solutions before and after illumination, wherein the solutions comprise (a) Cu(II)-TCPP with Co(NO₃)₂ at pH=7.0 ; (b) Cu(II)-TClTCPP with Co(NO₃)₂ at pH=7.0 ; (c) Ni(II)-TCPP with Co₄O₄ cubane at pH=7.0 ; (d) Ni(II)-TClTCPP with Co₄O₄ cubane at pH=7.0.

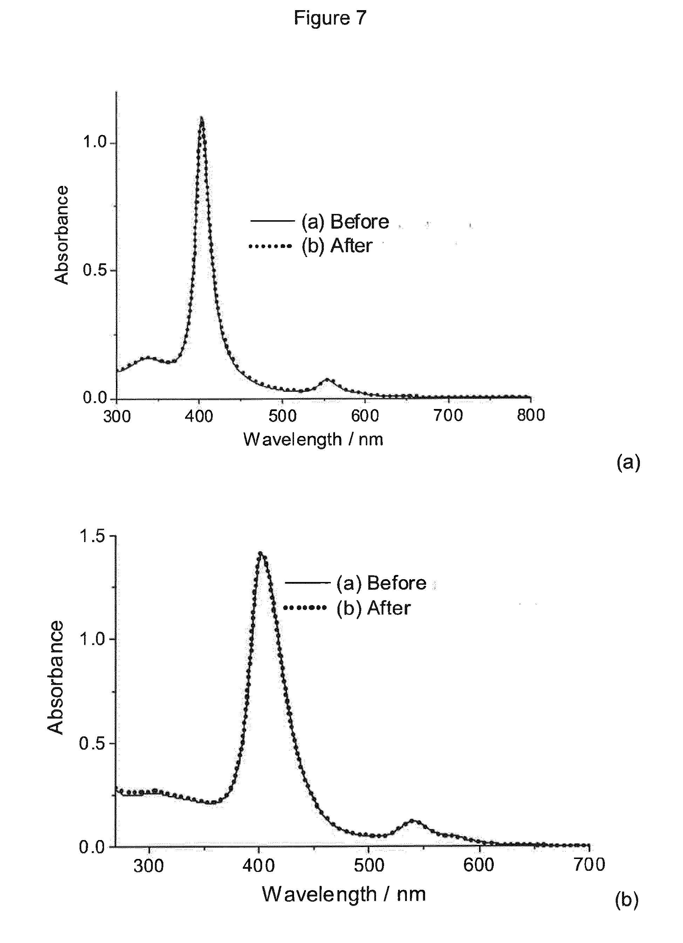

**Figure 7** provides UV-vis spectra, before and after illumination, of photocatalytic solutions of (a) Cu(II)-TClTCPP with Co(NO₃)₂ at pH=7.0 and (b) Ni(II)-TClTCPP with Co₄O₄ cubane at pH=7.0.

### DETAILED DESCRIPTION

As noted above, the present invention provides a photosensitizer comprising a porphyrin compound of the formula (I). Without wishing to be bound by any theory, it is believed that the electron withdrawing character of the halogen substituents in the halogenated porphyrins significantly influences the electrochemical properties of the porphyrinic derivatives. In particular, halogenation at the pyrrole β-positions of a porphyrin compound can provide a positive shift in oxidation potential, resulting in an improved ability to photoactivate the WOC in comparison to a corresponding, non-halogenated porphyrin. Moreover, the nonbonding electrons of the halogen atoms may also be conjugated to the π-conjugated porphyrin ring system, resulting in a red-shift in the absorption spectrum of halogenated porphyrins compared to an analogous nonhalogenated porphyrin. An improved light-harvesting ability can also be achieved.

Further, the halogenated porphyrins described herein are good candidates for use as a photosensitizer in water oxidation, given that they are inert in water and oxygen-saturated buffer solutions.

In the compound of the Formula (I) described herein, M may be any suitable metal. In one embodiment, the metal is selected from Ru, Cu, Ni, Mg, Zn, Co, Pd, Pt, Mn, Fe, Ag, Sn, Au, In, Ir, Ge, Pb, Al and Cr. In another embodiment, M may be Ru(II), Cu(II), Ni(II), Mg(II), Zn(II), Co(II), Pd(II), Pt(II), Mn(III), Fe(III), Sn(IV), Ag(II), Au(III), In(III), Ir(III), Ge(IV), Pb(II), Al(III), or Cr(III). For example, the metal M may be selected from Ru(II)(CO), Mn(III)Cl, Fe(III)Cl, Sn(IV)Cl₂, Mn(III)Cl, Fe(III)Cl, Ge(IV)Cl₂, AI(III)CI or Cr(III)Cl. Optionally, ligands CO and Cl may be axially coordinated to the central M atom.

In a preferred embodiment, the metal is an earth-abundant metal i.e. a metal that is abundant in the earth's crust. In a preferred embodiment, M is selected from Cu and Ni.

In the compound of the formula (I) described herein, M may alternatively be a non-metal selected from P and Si. For example, the non-metal and axially coordinating ligand may be selected from P(V)Cl₂ and Si(IV)Cl₂, wherein Cl is the optional, axially coordinated ligand.

In the compound according to the present invention, X may be any suitable halogen e.g. F, Cl, or Br. In one embodiment, each X is independently selected from Cl and F. In a preferred embodiment, each X is Cl. In another preferred embodiment, each X is F. In one preferred embodiment, the porphyrin structure is halogenated with four halogen atoms. For example, in the compound (I), X₁, X₂, X₃ and X₄ may be Cl and X₅, X₆, X₇ and X₈ may be H. In one embodiment, the porphyrin structure is halogenated with three halogen atoms. For example, in the compound (I), X₁, X₂ and X₃ may be Cl and X₄, X₅, X₆, X₇ and X₈ may be H. In a further embodiment, the porphyrin structure is halogenated with five halogen atoms. For example, in the compound according to structure (A), X₁, X₂, X₃, X₄ and X₅ may be Cl and X₆, X₇ and X₈ may be H.

In one embodiment, there is provided a compound having a chemical structure selected from: 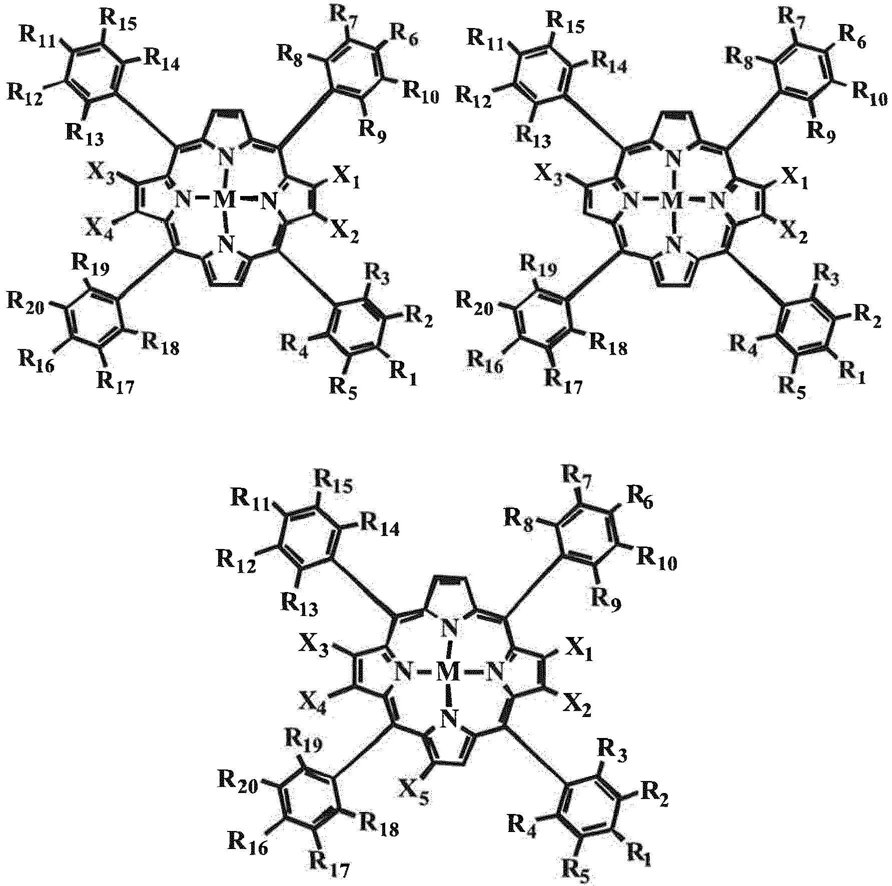 wherein each R is independently selected from the group consisting of: 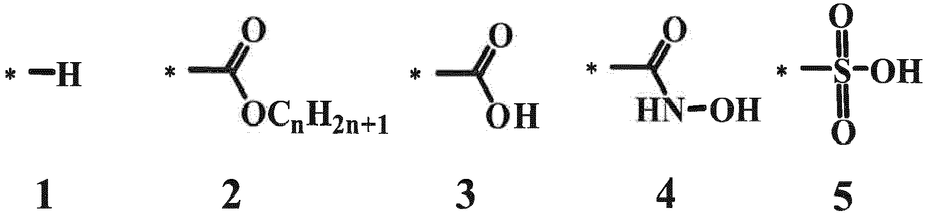 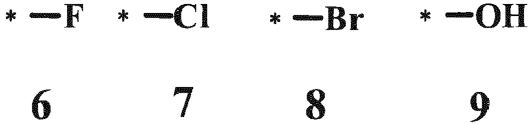 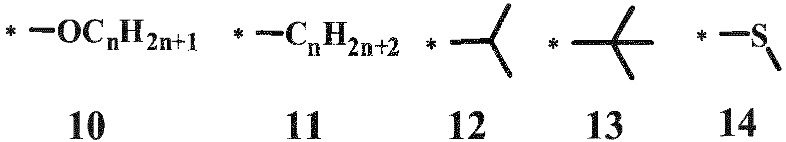 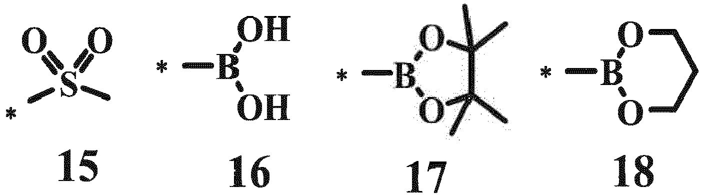 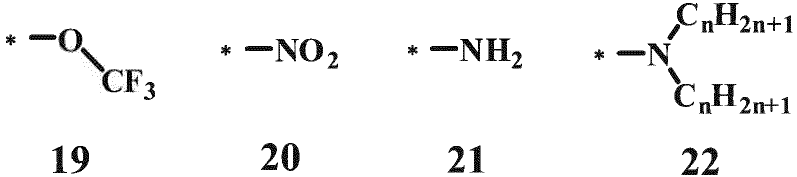 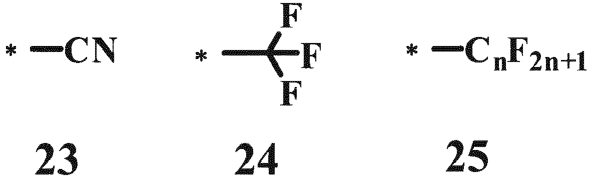 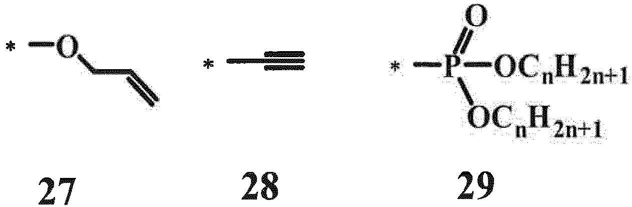 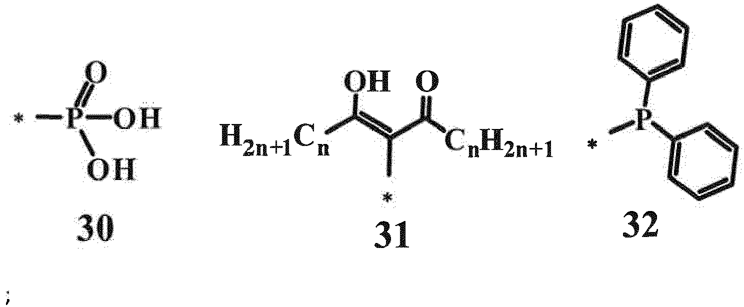 wherein each X above is a halogen, and
wherein M is as defined above.

In another embodiment of the present invention, the chemical structure may be selected from: 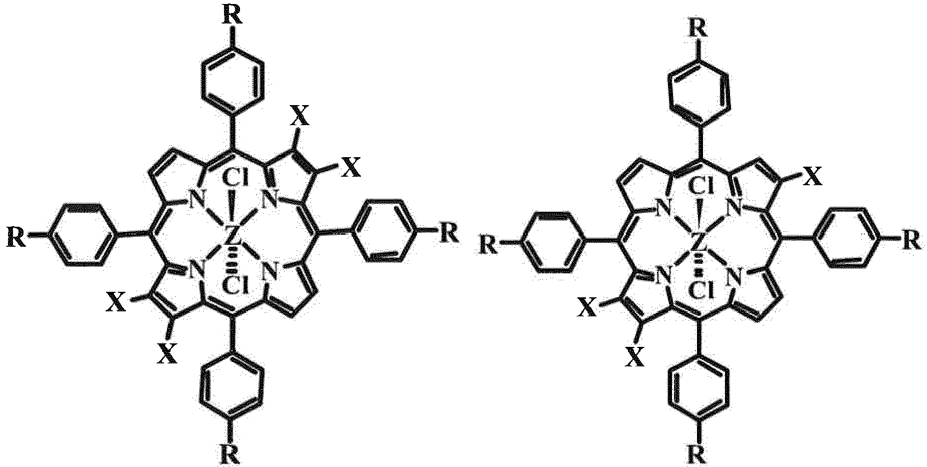 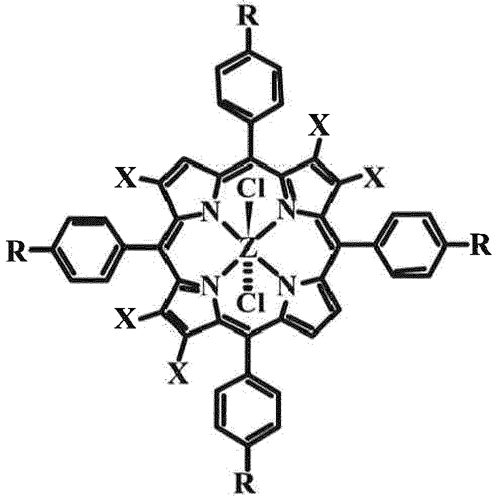 wherein Z is selected from P and Si;
each X is independently selected from halogen; and
and each R group is independently selected from:

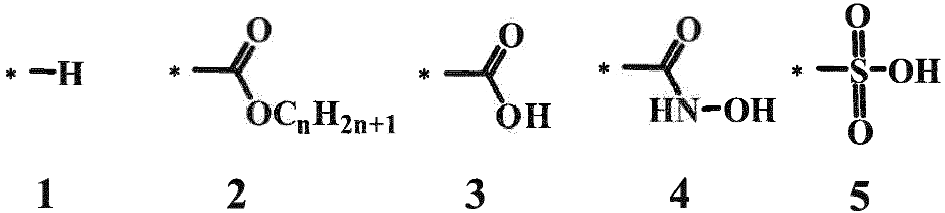

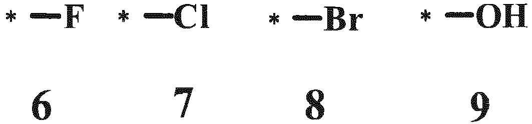

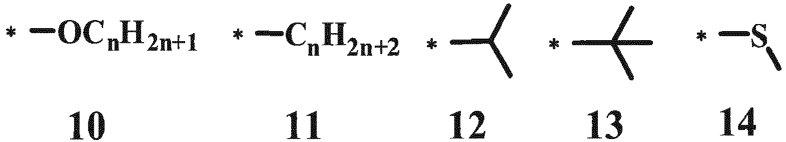

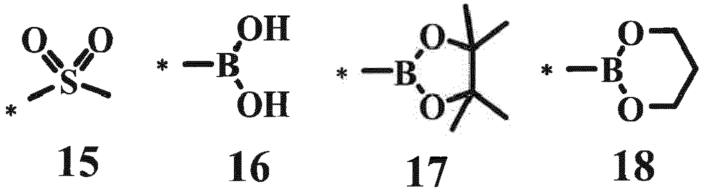

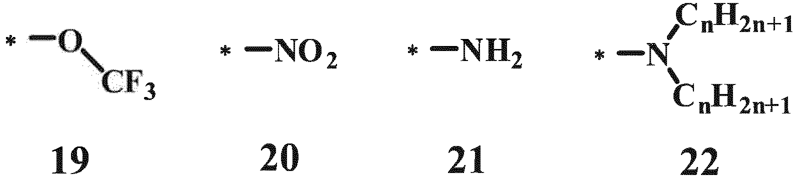

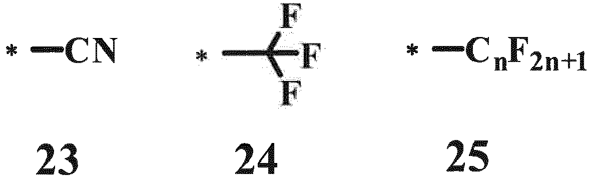

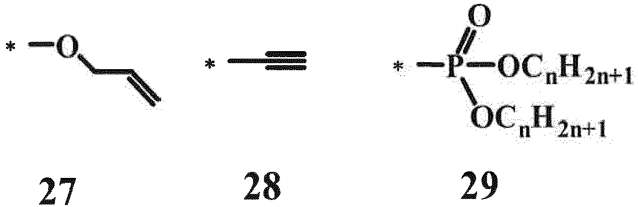

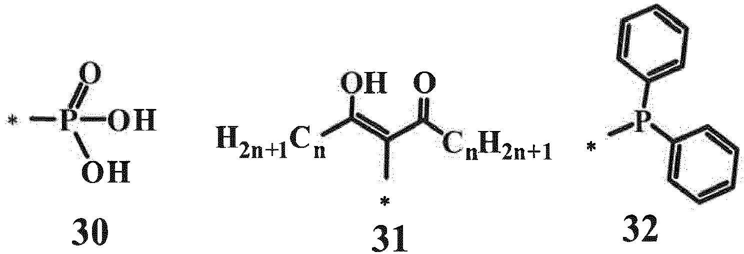

In one embodiment, compound (I) has the following structure: 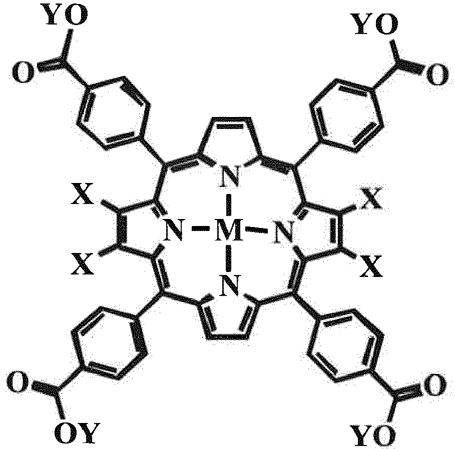 wherein M is selected from Cu or Ni, X is selected from Cl or F, and Y is selected from H or CH₃. In one embodiment, M is Cu, X is Cl and Y is CH₃. In another embodiment, M is Ni, X is Cl and Y is CH₃-In a further embodiment, M is Cu, X is Cl and Y is H.

In one embodiment, the porphyrin has the following structure:

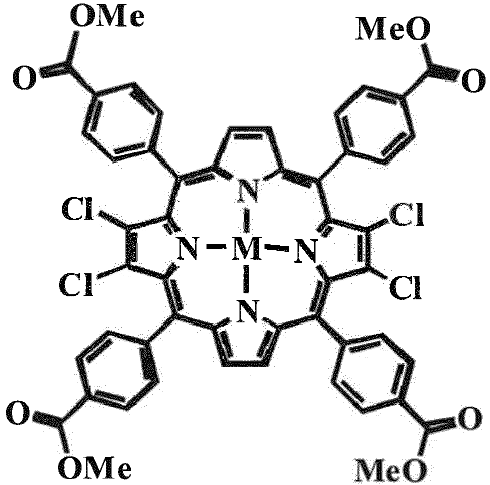

In the compound above, M may be selected from Cu or Ni. These compounds are Copper (II)-2,3,12,13-tetrachloro-5,10,15,20-tetrakis(4-methoxycarbonylphenyl)porphyrin (Cu(II)-TClTCMePP) and Nickel (II)-2,3,12,13-tetrachloro-5,10,15,20-tetrakis(4-methoxycarbonylphenyl)porphyrin (Ni(II)-TClTCMePP).

In another embodiment, the porphyrin has the following structure:

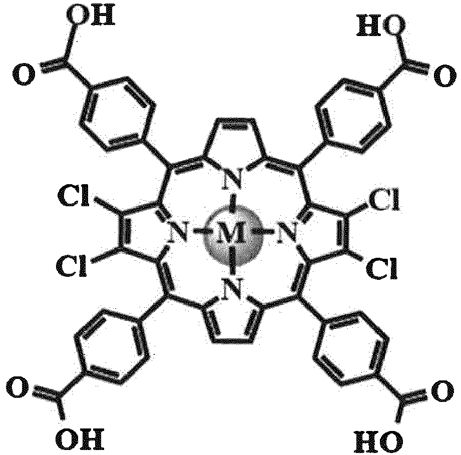

In the compound above, M is selected from Cu or Ni. These compounds are Copper (II)-2,3,12,13-tetrachloro-5,10,15,20-tetra(4-carboxyphenyl)porphyrin (Cu(II)-TClTCPP) and Nickel (II)-2,3,12,13-tetrachloro-5,10,15,20-tetra(4-carboxyphenyl)porphyrin (Ni(II)-TClTCPP).

For the avoidance of doubt, the compounds of the formula (I) may be derived from the corresponding "free-base" compounds in which M is absent and 2 H atoms are bonded to 2 of the N atoms in the porphyrin ring.

Below are examples of compounds of the formula (I) and their corresponding "free-base" compounds:

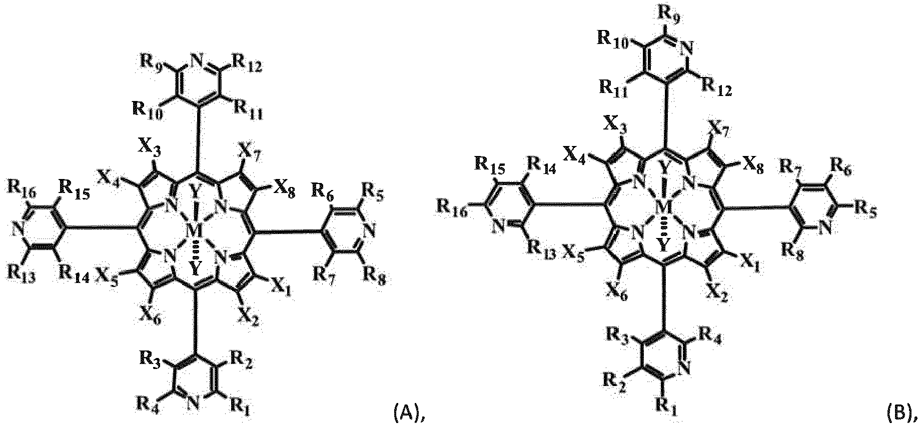

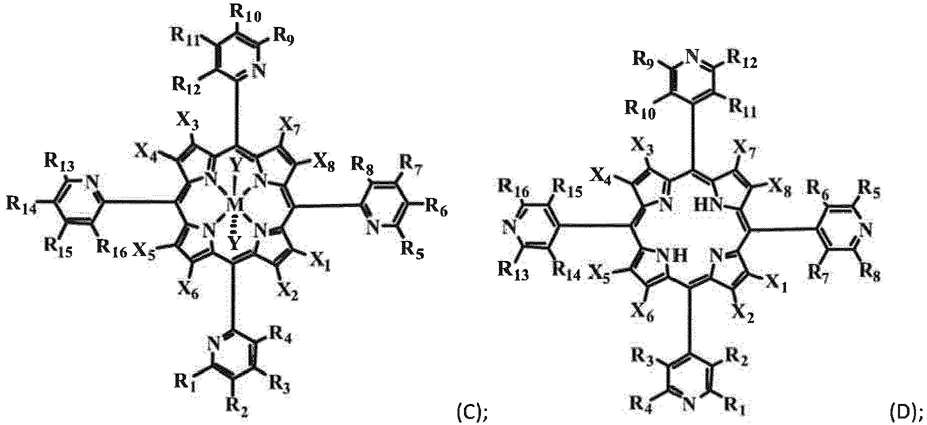

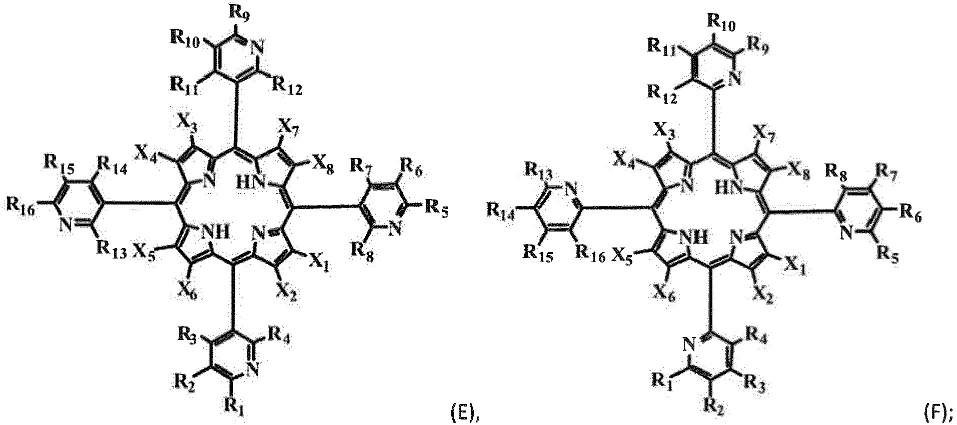

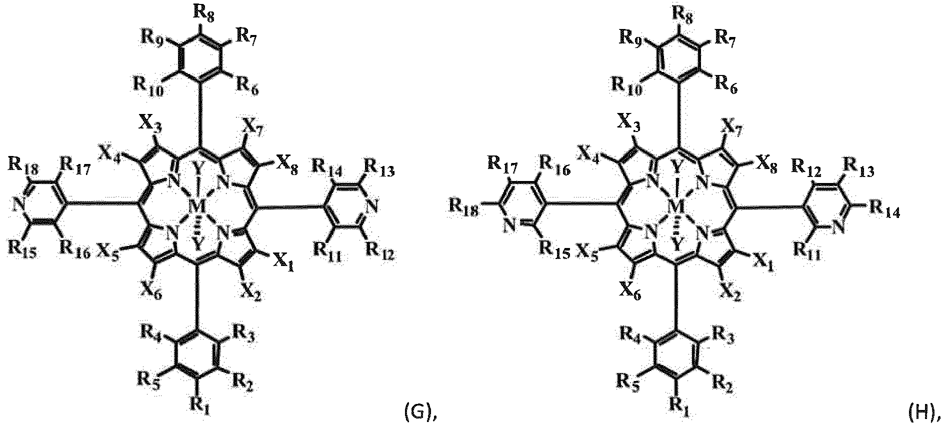

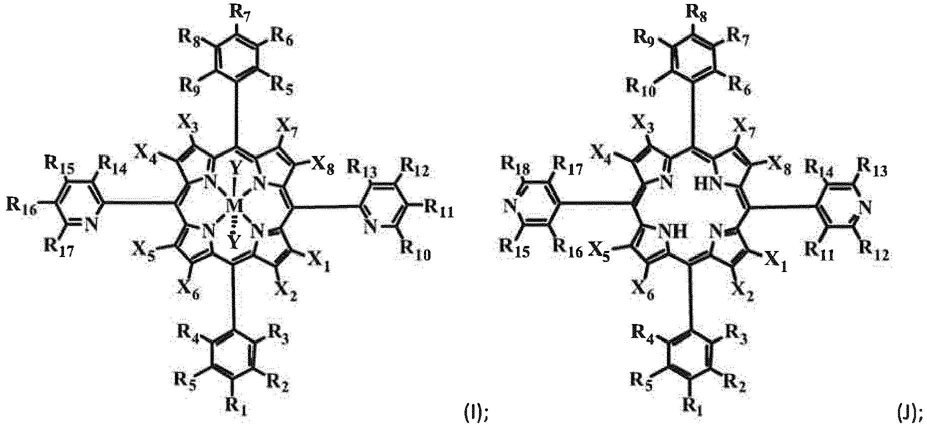

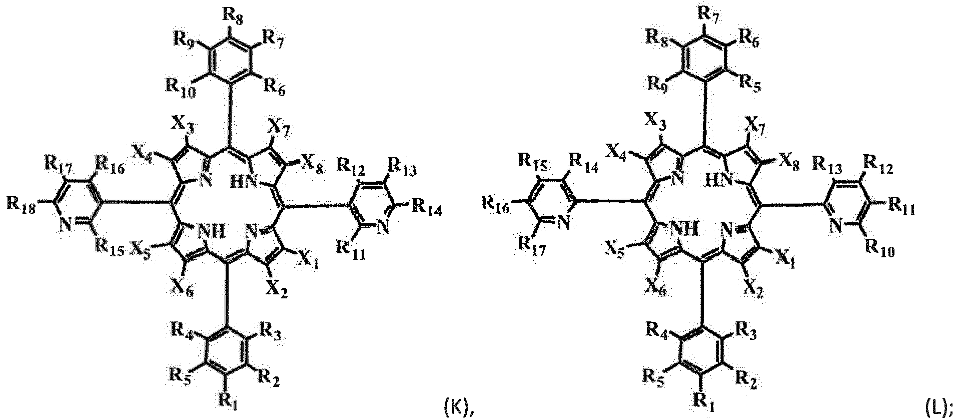

In the structures (A) to (L) above, each R is independently selected from:

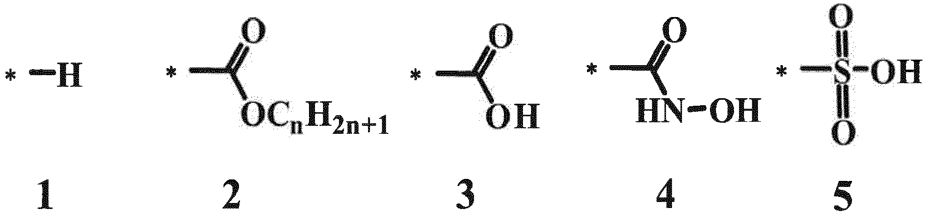

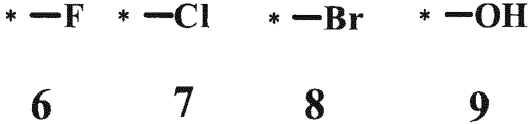

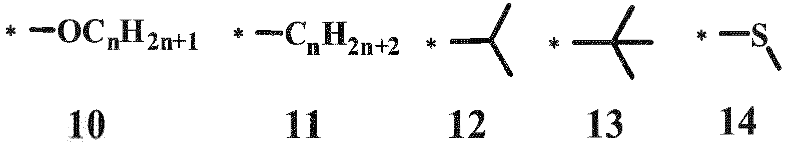

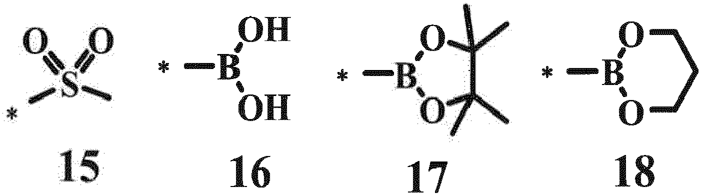

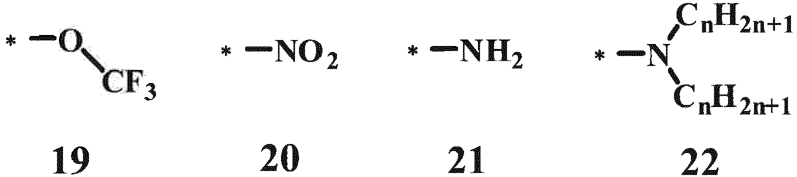

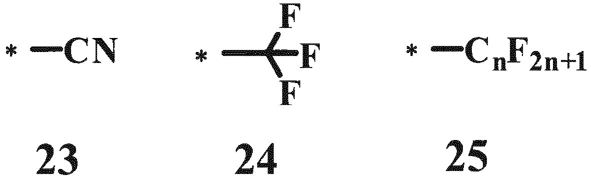

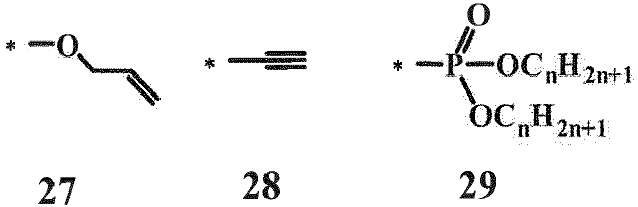

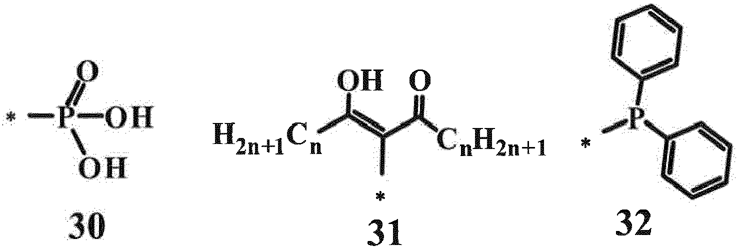

X₁ to X₈ and M are as defined above. Preferably, X₁ to X₄ are each halogen and X₅, X₆, X₇ and X₈ are each H; or X₅ and X₆ are linked together form an aromatic ring, and X₇ and X₈ are linked together form an aromatic ring.

Further examples of compounds of the formula (I) or their corresponding "free-base" compounds include:

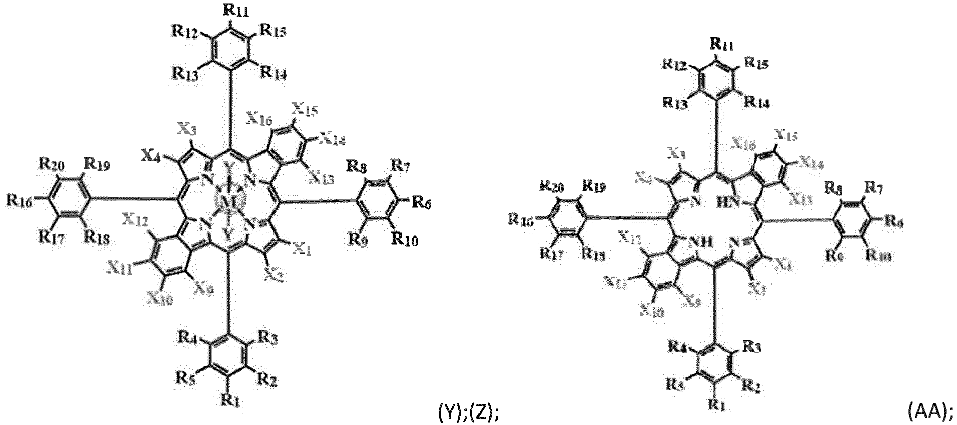

In the compounds above, each R is independently selected from:

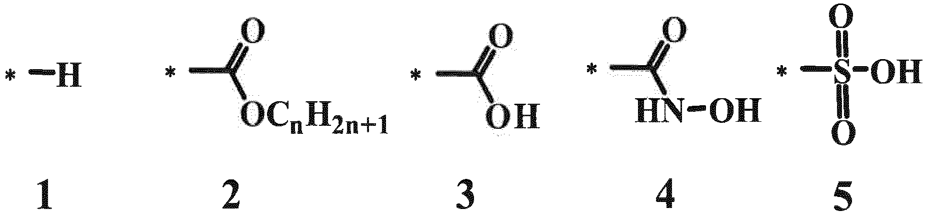

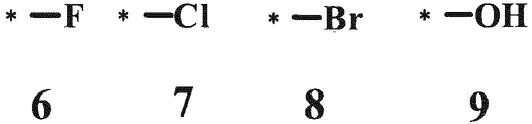

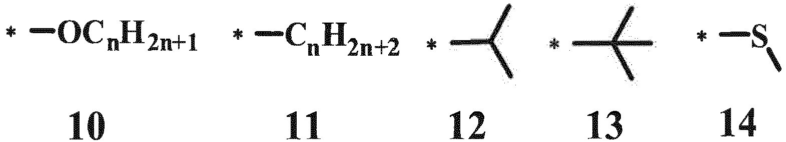

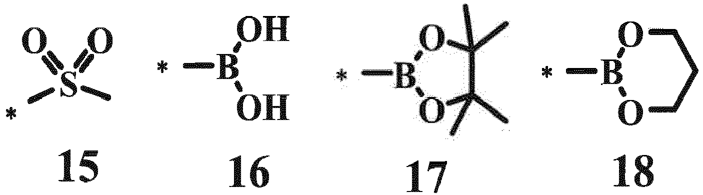

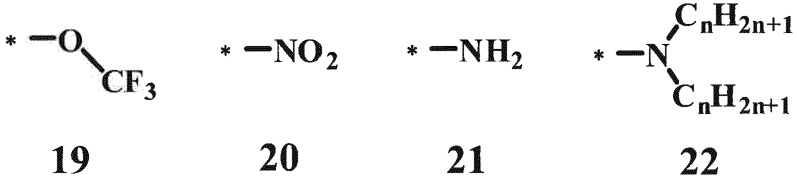

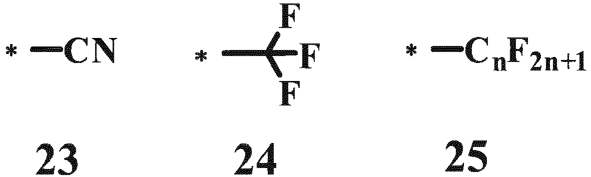

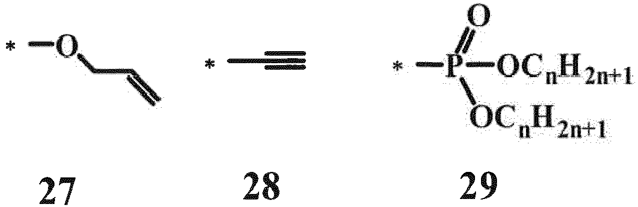

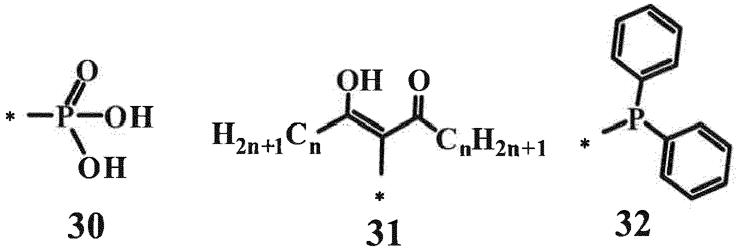

X₁ to X₄ and M are as defined above. In the structures above, X₅ and X₆ are linked together form a phenyl ring, and X₇ and X₈ are linked together form a phenyl ring. The phenyl rings are substituted by X₉ to X₁₆, which are each optionally selected from hydrogen, hydrocarbyl or halogen-containing group. Suitable hydrocarbyl groups include C₁ - C₁₀ hydrocarbyl groups, for example, C₁ - C₁₀ alkyl groups (e.g. methyl, ethyl, propyl and butyl). Suitable halogen-containing groups include halogens and halogenated hydrocarbyl groups. Suitable halogens include F, Cl and Br. Suitable halogenated hydrocarbyl groups include halogenated alkyl groups, for instance, halogenated C₁ - C₁₀ alkyl groups, Examples include -CₙX₂ₙ₊₁, where X is F, Cl, Br or I and n is an integer of 1 to 6.

As mentioned above, the photosensitizer described herein may be used in a system for performing visible-light-driven water oxidation. The system may comprise: the compound of Formula (I) above, a water oxidation catalyst (WOC); and a sacrificial electron acceptor. In one embodiment, the system further comprises water. The system enables the oxidation of water to be performed on exposure of the system to light of a suitable wavelength.

In one embodiment, the water oxidation catalyst may be an Ru, Ir, Mn, Co, Cu or Fe complex. The catalyst may be a mononuclear water oxidation catalyst. In another embodiment, the water oxidation catalyst may be a related microscale or nanoscale material, such as RuO₂, IrO₂, Co₃O₄, MnO₂, CoPi, CoCi. Examples of suitable water oxidation catalysts include CoOx, CoFeOₓ, IrOₓ, NiOₓ, NiCeOₓ, NiCoOₓ, NiCuOₓ, NiFeOₓ and NiLaOₓ.

In another embodiment, the water oxidation catalyst may be a related nanoscale layered double hydroxide (LDH) of Ni-Fe, Ni-Co Zn-Co, Co-Fe and Co-Mn.

In a preferred embodiment, the water oxidation catalyst is a Co₄O₄-cubane catalyst or a cobalt-oxide nanoparticle catalyst composed of Co(OH)ₓ. Co(NO₃)₂ may be employed as a pre-catalyst for a cobalt-oxide nanoparticle catalyst composed of Co(OH)ₓ. During photocatalysis of water, the Co²⁺ ion may be oxidized to form the cobalt-oxide nanoparticle.

As noted above, the system further comprises a sacrificial electron acceptor. The sacrificial electron acceptor may be any suitable compound that is capable of oxidizing the photosensitizer. In one embodiment, the sacrificial electron acceptor is selected from K₂S₂O₈, [Co(NH₃)₅Cl]Cl₂ and Na₂S₂O₈. In a preferred embodiment, the sacrificial electron acceptor is K₂S₂O₈.

The scheme below is a schematic illustration of the photo-oxidation of water by the three-component system described above. In the scheme below, the presence of the sacrificial electron acceptor containing S₂O₈²⁻ ions results in the formation of SO₄²⁻ ions.

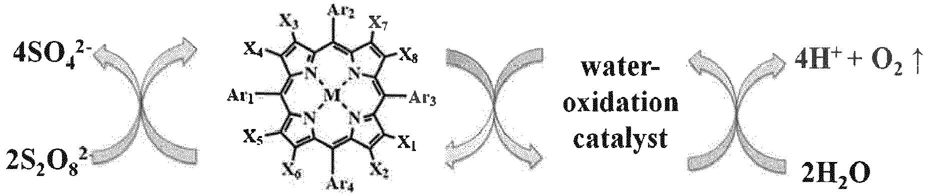

Preferably, the system according to the present invention comprises a buffer solution. Because proton-coupled electron transfer (PCET) plays a key kinetic role in catalytic water-oxidation mechanisms, buffer base and concentration may have a significant influence on the catalytic activity. Catalytic water oxidation may result in progressive acidification of the reaction medium, which results in less favourable thermodynamics of the system and a decrease in water oxidation activity. The presence of a buffer solution enables this acidification to be reduced or avoided.

Preferably, the buffer solution is selected from a phosphate, acetate, phthalate, borate and maleate buffer solution. In a preferred embodiment, the buffer solution is a phosphate buffer, for example potassium phosphate.

In one embodiment, the buffer solution has a pH of between 4 and 10, preferably between 6 and 7. In one example, the pH of the buffer solution is approximately 7.

As noted above, the photosensitizer described herein may be used to form a photoelectrode, for example, a photoanode. The photoanode comprises a current collector, a semiconductor metal oxide coating, a photosensitizer as described above, and, optionally, a water oxidation catalyst. The photoanode may comprise a transparent conducting electrode (TCE) as the current collector. In one embodiment, the transparent conducting electrode is selected from indium tin oxide (ITO) or fluorine-doped tin oxide (FTO). The electrode may be coated with a semiconductor metal oxide by any suitable method. In one embodiment, the metal oxide MₓO_{y} is selected from TiO₂, SnO₂, and MgO passivated SnO₂.

The photoanode further comprises the photosensitizer compound described above, and a water oxidation catalyst as described above.

The photosensitizer and WOC may form a single layer, or may form two separate layers. The photosensitizer and WOC layer or layers may be applied to the metal oxide coating by any suitable method. In some embodiments, the photosensitizer and WOC may be applied by co-adsorption or layer-by-layer stacking. In one embodiment, the photosensitizer and WOC are applied by layer-by-layer stacking, wherein the WOC is applied on the top of the photosensitizer layer. In one embodiment, the photosensitizer for photoanode fabrication is a compound according to compound (I) above.

In a preferred embodiment, the photoanode is formed by co-deposition. In one embodiment, the photoanode may be formed by the by (a) co-deposition or (b) layer-by-layer stacking of the photosensitizer and WOC on the metal oxide semiconductor layer, as shown in Figure 2. For example, the photoanode may be formed by co-deposition of the photosensitizer with an Ir-NHC water WOC, as shown in Figure 3.

In an alternative embodiment, the porphyrin is a Si(IV)Cl₂ or P(V)Cl₂ porphyrin, wherein Cl is the axially coordinated ligand. The porphyrin can be arranged as a multilayer structure. In one embodiment, the Si(IV)-porphyrin can be cofacially aligned by a siloxane bridge linker and attached to the electrode. In one embodiment, the Si(IV)-porphyrin is attached to the electrode by Si-O covalent bonding. In a preferred embodiment, the Si(IV)Cl₂-porphyrin photosensitizer is immobilized on a transparent conducting electrode coated with a semiconductor metal oxide by Si-O covalent bonding in water. In one embodiment, a Si(IV)Cl₂-porphyrin photosensitizer is directly immobilized on transparent conducting electrode by Si-O covalent bonding in water.

In one embodiment of the invention, the WOC may be applied by co-adsorption alongside the photosensitizer, or by layer-by-layer stacking (WOC on the top of the photosensitizer layer). Figure 4 provides an illustration of co-absorption ((a) and (b)) or layer-by-layer stacking ((c) and (d)). The photosensitizer layer can comprise either a single porphyrin, or multilayer porphyrins. In one embodiment, the multiple porphyrins are cofacially aligned. Any number of porphyrins may form the multilayer. In a preferred embodiment, the multilayer is made up of between 2 and 100 porphyrin layers. In a preferred embodiment, the photosensitizer comprises cofacially aligned Si(IV)-porphyrins. In one embodiment, the multilayer Si(IV)-porphyrins are homo- or hetero-Si(IV)-porphyrin cofacially aligned.

The present disclosure further provides a cell for light-driven water oxidation. The cell comprises a photoanode as described herein, a photocathode, and an electrolyte. The photosensitizer described herein may be present in the photoanode or, if water-soluble, dissolved in the electrolyte.

Any suitable electrolyte may be employed in the cell. In one embodiment, the electrolyte comprises a buffer solution. The electrolyte is dependent on the pH. Preferably, the buffer solution is selected from a phosphate, acetate, phthalate, borate and maleate buffer solution. In a preferred embodiment, the buffer solution is a phosphate buffer, for example potassium phosphate.

As noted above, the present invention also provides a method of oxidizing water (i) providing a cell described herein; (ii) introducing water into the cell; and (iii) exposing the cell to light of a suitable wavelength. During the oxidation of water, oxygen is produced at the photoanode, and protons are reduced to produce hydrogen at the photocathode.

The light may be of any suitable wavelength. In a preferred embodiment, the light may be any wavelength in the solar spectrum. In one embodiment, the light may be infrared, visible or ultraviolet light. In a preferred embodiment, the light is visible light. In one embodiment, the light has a wavelength of 300 nm to 1000 nm. In one embodiment, the light source is a solar simulator. In another embodiment, the light source is sunlight.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

The synthesis of a compound of the formula (I) will now be described with reference, by way of example, to 5,10,15,20-tetrakis(4-methoxycarbonylphenyl)porphyrin. High-yield synthesis (up to 80%) of chlorinated Cu(II) and Ni(II) derivatives of 5,10,15,20-tetrakis(4-methoxycarbonylphenyl) porphyrin, with four halogens at β-pyrrole positions, was performed via a modification of a known halogenation approach (P. Ochsenbein, K. Ayougou, J. Fischer, R. Weiss, N. Rachel, K. Jayaraj, A. Gold, J. Terner, and J. Fajer, Angew. Chem. Int. Ed., 1994, 33, 348-350). In step (1), the free-base porphyrin is reacted with 5.5 equivalent (mole.) N-chlorosuccinimide (NCS) in a high boiling point chlorohydrocarbon solvent (1,2-dichloroethane) at 100°C overnight under nitrogen condition. In step (2), halogenated metalloporphyrins were synthesized using known metal insertion procedures (A. D. Adler, F. R. Longo, F. Kampas, and J. Kim, J. Inorg. Nucl. Chem., 1970, 32, 2443-2445). The reagents (halogenated porphyrin and metal chloride or metal acetate, metal acetate is preferred) are dissolved in dimethylformamide (DMF) and heated at 140°C under nitrogen conditions. In step (3), ester hydrolysis was performed using known procedures (R. P. Briñas, T. Troxler, R. M. Hochstrasser, and S. A. Vinogradov, J. Am. Chem. Soc., 2005, 127, 11851-62). The chlorinated metallo-5,10,15,20-tetrakis(4-methoxycarbonylphenyl)porphyrin was reacted with dilute alkali solution (potassium hydroxide) at room temperature.

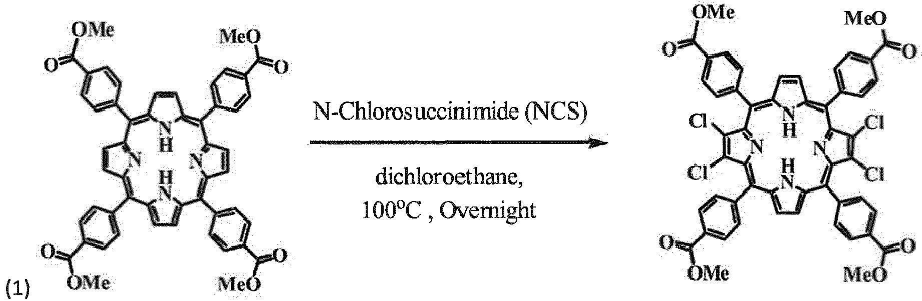

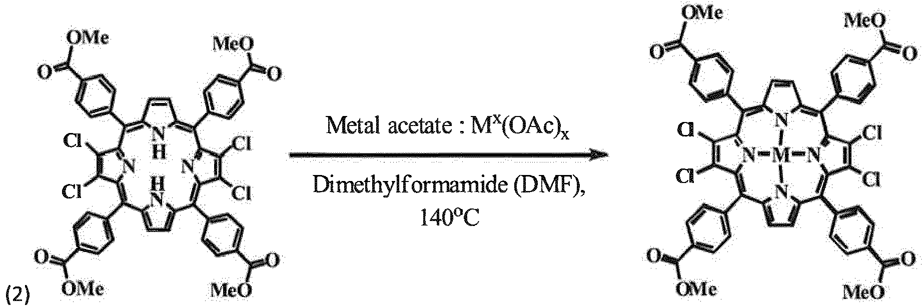

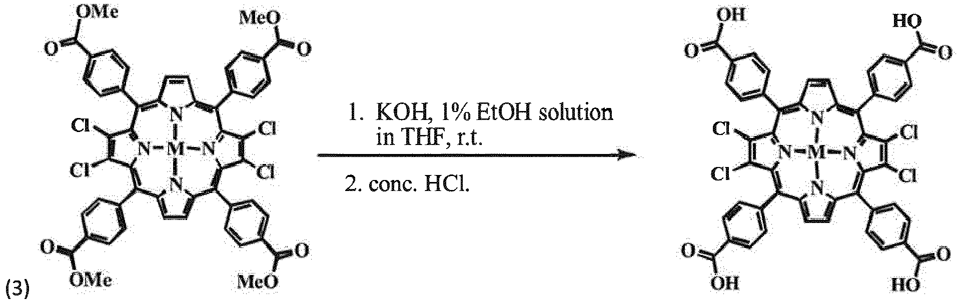

### EXAMPLES

Light-driven water oxidation studies were performed on photosensitizers having the following structures.

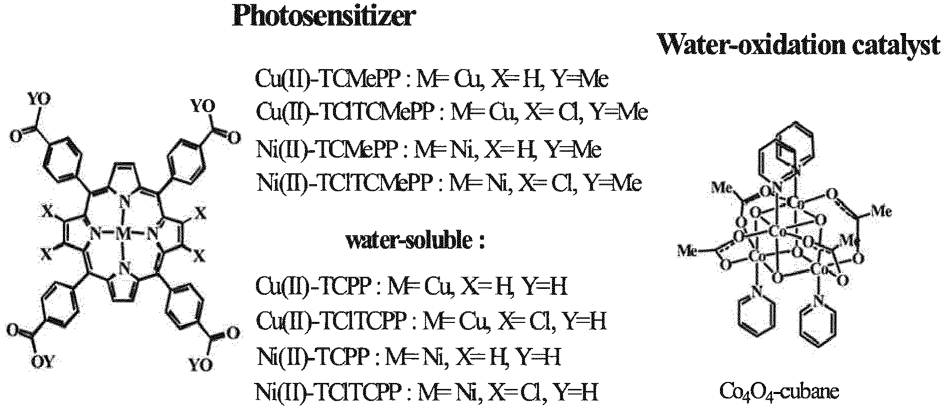

The light-driven water oxidation studies were performed in a concentrated (0.1 M KPi) phosphate buffer solution. The studies focussed on a three-component system composed of (a) a photosensitizer as listed above, (b) K₂S₂O₈ as the sacrificial electron acceptor, and (c) either the Co-based water oxidation catalyst shown above (Co₄O₄-cubane) or Co(NO₃)₂ as a precatalyst as discussed above.

### Water oxidation

Photochemical oxygen generation was recorded by a Clark oxygen-electrode under 120W halogen lamp illumination. The O₂ generation in this photocatalytic system is believed to follow the well-established reaction mechanism shown below:

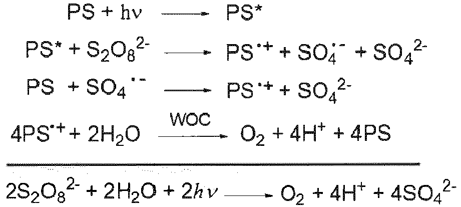

The results of a light control experiment (shown in Figure 5) shows that the catalytic oxidation of water in the presence of a photosensitizer according to the invention and Co(NO₃)₂ precatalyst is driven by visible light. The increase in O₂ yield is observed over several hours, indicating that the halogenated metalloporphyrins are highly photostable over this period. The slight [O₂] decrease observed during the "off" period results from equilibration of dissolved oxygen with oxygen in the headspace of the chamber in the Clark electrode.

Figure 6 shows the photocatalytic solutions before and after illumination. Figures 6a and 6c illustrate the change in colour of the nonhalogenated metalloporphyrins Cu(II)-TCPP and Ni(II)-TCPP following illumination with visible light. This indicates that decomposition of Cu(II)-TCPP and Ni(II)-TCPP has occurred. In contrast, Figures 6b and 6d show that, for the halogenated metalloporphyrins Cu(II)-TClTCPP and Ni(II)-TClTCPP, no colour change is observed, demonstrating the photostability of these photosensitizers over the same time period. The UV-vis spectra of photocatalytic solutions of Cu(II)-TClTCPP with CO(NO₃)₂ at pH=7.0 and Ni(II)-TClTCPP with Co₄O₄ cubane at pH=7.0 shown in Figure 7 before and after illumination further illustrate the photostability of these photosensitizers.

Table 1 provides integrated molar absorptivities and percentages of photons absorbed by a range of 2µM chromophore solutions (1 cm path length). The values for molar absorptivity for both Cu(II)-TClTCMePP and Ni(II)TClTCMePP are at least five times larger than those of a commonly used photosensitizer Ru(bpy)₃²⁺ because they show much more intense absorption in the visible light range. The 50% photon capture threshold (PCT⁵⁰) also provides an indication of the photo-absorption abilities of a chromophore, and represents the concentration of chromophore needed to absorb 50% of incident solar photons in the given solar spectrum range. The PCT⁵⁰ of Cu(II)-TClTCMePP and Ni(II)-TClTCMePP are about one order of magnitude smaller than that of Ru(bpy)₃²⁺. The PCT⁵⁰ is also only approximately a quarter of that of Pt(II)-TCMePP, due to the broadening and red-shift of absorption provided by the halogenation in Cu(II)-TClTCMePP and Ni(II)-TClTCMePP. The photon absorption is also shown to be higher than that of chlorophyll *a*. Even though chlorophyll *a* has broader transitions that span a comparatively large portion of the sunlight spectrum, the PCT⁵⁰ of the tetrachloro-metalloporphyrins is still comparable to that of chlorophyll *a*.

Table 1 also provides oxidation potentials and shows, in particular, a higher oxidation potential for Cu(II)-TClTCMePP and Ni(II)-TClTCMePP in comparison to Ru(bpy)₃²⁺. The higher oxidation potential provides a larger driving force for electron transfer from the WOC to the radical cation of the photosensitizer. Importantly, the halogenated metalloporphyrins according to the present invention shown similar oxidation potentials and better photo capture abilities to the noble-metal non-halogenated Pt(II)-TCMePP.

**Table 1. Photo-absorption properties^{a} of representative chromophores referenced to AM1.5G solar irradiance photon flux and related oxidation potential**

| Molecule | integrated molar absorptivity (M⁻¹) | AM1.5G photon capture (2µM) | 50% photon capture threshold (PCT⁵⁰) | oxidation potential V vs NHE |
|---|---|---|---|---|
| Cu(II)-TClTCMePP | 6.5 × 10⁸ | 9.6% | 32 µM | 1.43 |
| Ni(II)-TClTCMePP | 7.0 × 10⁸ | 10.3% | 31 µM | 1.51 |
| Pt(II)-TCMePP | 4.5 × 10⁸ | 6.0% | 122 µM | 1.50 |
| Ru(bpy)₃²⁺ | 1.2 × 10⁸ | 1.7% | 360 µM | 1.26 |
| Chlorophyll *a* | 4.8 × 10⁸ | 7.2% | 38 µM | 0.81 |
| P680 | | | | 1.26 |

| | | | | |
|---|---|---|---|---|
| ^{a} 300 ∼ 730 nm. | | | | |

## Claims

1. A photosensitizer comprising a porphyrin compound of the formula (I): 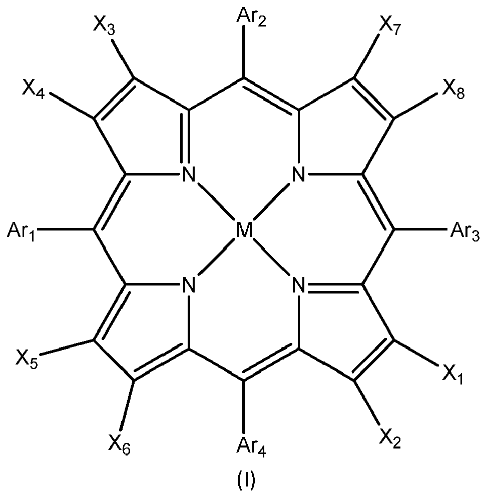 wherein:
Ar₁, Ar₂, Ar₃ and Ar₄ are each independently selected from aryl or heteroaryl, wherein each of said aryl or heteroaryl substituents may be optionally substituted;
X₁, X₂, X₃, and X₄ are each independently selected from H or halogen, whereby at least one of X₁, X₂, X₃, and X₄ is halogen;
X₅ and X₆ are each independently selected from H, hydrocarbyl or a halogen-containing group, or are linked together form an aromatic ring;
X₇ and X₈ are each independently selected from H , hydrocarbyl or a halogen-containing group, or are linked together form an aromatic ring; and
M is a metal, P or Si.

2. A photosensitizer as claimed in claim 1, wherein X₁, X₂, X₃, and X₄ are halogen.

3. A photosensitizer as claimed in claim 1 or 2, wherein
X₅, X₆, X₇ and X₈ are each H; or
X₅ and X₆ are linked together form an aromatic ring, and X₇ and X₈ are linked together form an aromatic ring.

4. A photosensitizer as claimed in any one of the preceding claims, wherein the aromatic ring comprises a phenyl ring that is fused to the pyrrole rings of the porphyrin.

5. A photosensitizer as claimed in any one of the preceding claims, wherein Ar₁, Ar₂, Ar₃ and Ar₄ each independently comprises a phenyl group or a pyridyl group.

6. A photosensitizer as claimed in any one of the preceding claims, wherein Ar₁, Ar₂, Ar₃ and Ar₄ are each independently selected from aryl or heteroaryl, wherein each of said aryl or heteroaryl substituent may be optionally substituted with a substituent comprising at least one halogen, hydrocarbyl, carboxylate, ketone, ether, ester, hydroxyl, sulphonate, sulphone, thioether, boron-containing group, amine, amide, nitrile, nitro, heteroalkyl, and phosphorus-containing group, in particular wherein Ar₁, Ar₂, Ar₃ and Ar₄ are each independently selected from aryl or heteroaryl, wherein said aryl or heteroaryl may be optionally substituted with a carboxylate group.

7. A photosensitizer as claimed in any one of the preceding claims, wherein M is selected from one of Cr, Ru, Ni, Mg, Zn, Co, Pd, Pt, Mn, Fe, Ag, Sn, Au, In, Ir, Ge, Pb, Al, Cu, P and Si.

8. A photosensitizer as claimed in claim 7, wherein M is Cu or Ni.

9. A photosensitizer as claimed in any one of the preceding claims, wherein M is co-ordinated to at least one further ligand.

10. A photosensitizer as claimed in any one of the preceding claims, wherein the halogen is selected from F and Cl.

11. A photosensitizer as claimed in any one of the preceding claims, wherein the compound (I) is of the formula (II) or a salt thereof: 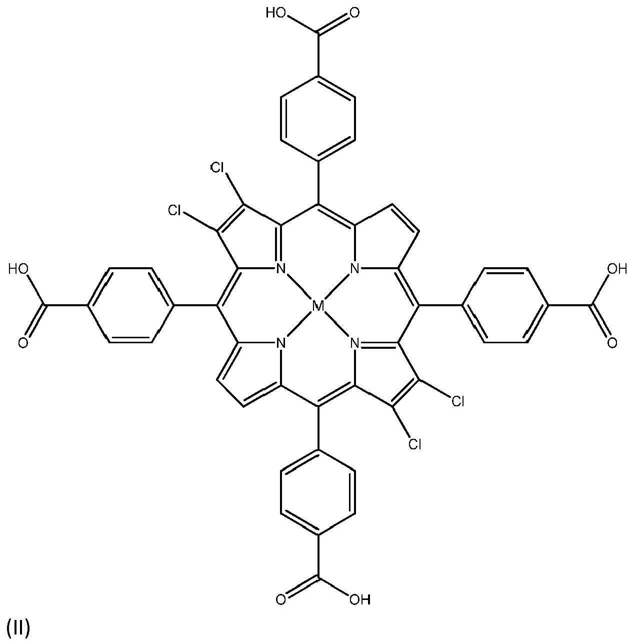 wherein M is Cu or Ni.

12. A photoelectrochemical cell comprising a photosensitizer as claimed in any one of the preceding claims, wherein the cell preferably comprises a) an electrolyte comprising the compound of the formula (I) dissolved or dispersed therein or b) a photoelectrode comprising a compound of the formula (I).

13. A photoelectrode comprising a photosensitizer as claimed in any one of claims 1 to 11.

14. A compound of the formula (I') or (I"): 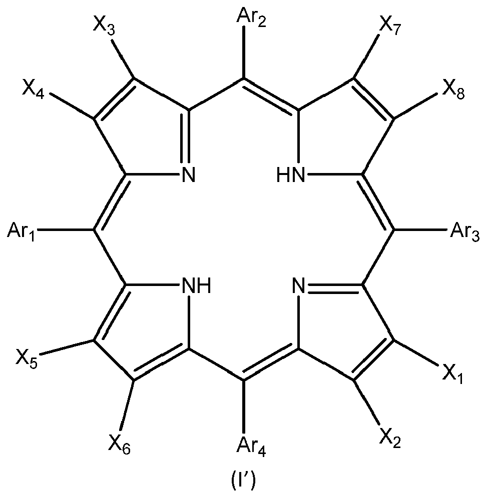 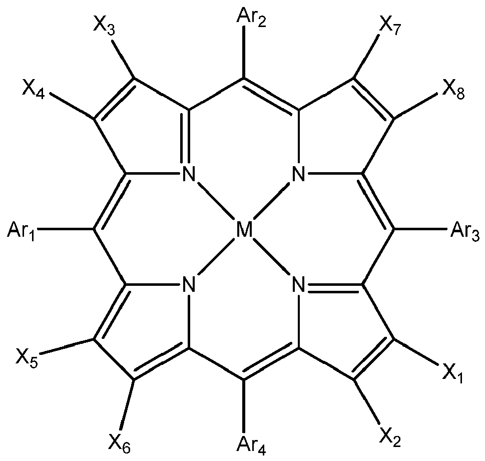 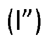 wherein:
Ar₁, Ar₂, Ar₃ and Ar₄ are each independently selected from aryl or heteroaryl, wherein each of said aryl or heteroaryl substituents may be optionally substituted,
X₁, X₂, X₃, and X₄ are each halogen,
X₅, X₆, X₇ and X₈ are each H; or
X₅ and X₆ are linked together form an aromatic ring, and X₇ and X₈ are linked together form an aromatic ring, and
where present, M is a metal, P or Si.

15. A compound as claimed in claim 14, which has the formula:
